# EUROPEAN PATENT APPLICATION

(11) **EP 3 888 722 A1**
(43) Date of publication of application: **06.10.2021**
(21) Application number: 19889584.9
(22) Date of filing: 26.11.2019
(51) Int. Cl.: A61M 5/158, A61M 25/06

(54) **INDWELLING NEEDLE ASSEMBLY**

(30) Priority: 29.11.2018 JP 2018223112; 04.03.2019 JP 2019038991
(71) Applicant: Nipro Corporation, Osaka-shi, Osaka 531-8510 (JP)
(72) Inventor: KUDO, Tatsuya, Osaka-shi, Osaka 531-8510 (JP); SHIBUYA, Makoto, Osaka-shi, Osaka 531-8510 (JP); UCHIMURA, Tomohiro, Osaka-shi, Osaka 531-8510 (JP); YOKOTA, Hiroki, Osaka-shi, Osaka 531-8510 (JP)
(74) Representative: Slingsby Partners LLP
(86) International application number: PCT/JP2019/046247
(87) International publication number: WO 2020/111080

(57) **Abstract**

An indwelling needle assembly 10 wherein an inner needle 22 is inserted through an outer needle 16 arranged to project to a distal end of a housing 26, and the inner needle 22 is extracted from the outer needle 16 by means of an urging member 122 and is housed in the housing 26 by pressing an operating member 28 provided to be exposed on an outer circumferential surface 27 of the housing 26. In a delivery operating part 18, which receives an operation force that delivers the outer needle 16 to the distal end from the housing 26, a lock part 42, 118 is provided which blocks movement of the operating member 28 in a direction of pressing while releasing blocking of the movement through delivery of the outer needle 16 by means of the delivery operating part 18. Furthermore, a positioning mechanism 42, 44, 100, 118 is provided which positions the delivery operating part 18 with respect to the operating member 28 in the circumferential direction of the housing 26.

## Description

### TECHNICAL FIELD

The present invention relates to an indwelling needle assembly provided with an outer needle that is stuck and indwelled when performing infusion, blood collection, hemodialysis, and the like, and particularly to an indwelling needle assembly capable of housing an inner needle extracted after sticking.

### BACKGROUND ART

Conventionally, an indwelling needle assembly has been known as a medical device for performing treatments such as infusion, blood collection, hemodialysis, and the like. Examples of such an indwelling needle assembly include those described in Japanese Unexamined Patent Publication No. JP-A-2015-047493 (Patent Document 1). The indwelling needle assembly described in Patent Document 1 has a structure in which an inner needle is inserted in an outer needle. After the inner and outer needles are stuck into a blood vessel of a patient, the inner needle is pulled out of the outer needle, such that the outer needle is indwelled in the blood vessel of the patient and used for treatment.

Meanwhile, in the indwelling needle assembly described in Patent Document 1, when the inner needle is pulled out of the outer needle, the urging force of an urging member housed within the housing is utilized. That is, in the state before the indwelling needle assembly is stuck, an operating member provided on the outer circumferential surface of the housing and an inner needle hub that fixedly supports the inner needle are engaged, and the inner needle is maintained in a projecting state from the housing against the urging force of the urging member. Then, after sticking, through pressing operation of the operating member, the engagement between the operating member and the inner needle hub is released, and the inner needle moves in the direction of being pulled out of the outer needle according to the urging force of the urging member, so as to be housed in the housing.

### BACKGROUND ART DOCUMENT

### PATENT DOCUMENT

Patent Document 1: JP-A-2015-047493

### SUMMARY OF THE INVENTION

### PROBLEM THE INVENTION ATTEMPTS TO SOLVE

However, in the indwelling needle assembly described in Patent Document 1, for example, even in the state before sticking, the inner needle may be pulled out of the outer needle by the operating member being unintentionally pushed down.
It may be an object of the present invention to provide an indwelling needle assembly having a novel structure capable of preventing an unintended pressing operation of an operating member.

Moreover, in the indwelling needle assembly described in Patent Document 1, when the inner needle moves in the direction of being pulled out of the outer needle according to the urging force of the urging member and is housed in the housing, a large impact sound may be generated to cause discomfort feeling to the user.
It may be an object of the present invention to provide an indwelling needle assembly having a novel structure in which an impact sound when the inner needle is housed in the housing is quiet.

### MEANS FOR SOLVING THE PROBLEM

A first preferred embodiment of the present invention provides an indwelling needle assembly including: a housing; an outer needle arranged so as to project to a distal end side of the housing; and an inner needle inserted through the outer needle, the inner needle being configured to be housed in the housing by means of an urging member through pressing operation of an operating member that is provided so as to be exposed on an outer circumferential surface of the housing, wherein the indwelling needle assembly further includes: a delivery operating part configured to receive an operating force that delivers the outer needle from the housing to the distal end side; a lock part provided with respect to the delivery operating part, the lock part blocking movement of the operating member in a direction of pressing while releasing blocking of the movement through delivery of the outer needle by means of the delivery operating part; and a positioning mechanism that positions the delivery operating part with respect to the operating member in a circumferential direction of the housing.

According to the indwelling needle assembly structured following the present preferred embodiment, the sliding operation of the inner needle by the operating member can be blocked by the lock part until the outer needle is delivered, which is generally performed after sticking. Therefore, the inner needle is prevented from being housed in the housing by, for example, pushing down the operating member unintentionally before sticking. In addition, since the block by the lock part is released by skillfully utilizing the delivery operation of the outer needle, which is generally performed after sticking, no special operation for unlocking is required, thereby obtaining good operability.

Besides, with the indwelling needle assembly according to the present preferred embodiment, the delivery operating part is positioned in the circumferential direction with respect to the operating member. This may prevent an accidental rotation of the delivery operating part, thereby more stably performing the delivery operation of the delivery operating part, for example.

A second preferred embodiment of the present invention provides the indwelling needle assembly according to first preferred embodiment, wherein the delivery operating part is constituted by an outer needle hub provided on a proximal end side of the outer needle.

The indwelling needle assembly structured following the present preferred embodiment makes it possible to directly deliver the outer needle hub provided to the outer needle.

A third preferred embodiment of the present invention provides the indwelling needle assembly according to the first preferred embodiment, wherein the delivery operating part is constituted by a slider arranged between an outer needle hub provided on a proximal end side of the outer needle and the housing.

According to the indwelling needle assembly structured following the present preferred embodiment, the outer needle can be delivered by operating the slider. Thus, for example, the delivery operation can be performed while avoiding contact with the outer needle including the outer needle hub, and the degree of freedom in designing the delivery operating part constituted by the slider can also be improved while avoiding an influence on the outer needle including the outer needle hub. Furthermore, since the outer needle can be pushed out without touching the outer needle hub with a finger, it is possible to prevent the outer needle hub from being contaminated due to undesired contact.

A fourth preferred embodiment of the present invention provides the indwelling needle assembly according to any of the first through third preferred embodiments, wherein a support mechanism is provided, the support mechanism allowing delivery of the delivery operating part to the distal end side while coaxially positioning the delivery operating part with respect to the housing, and the lock part is constituted by a structure in which the operating member and the delivery operating part are in contact with each other in an axis-perpendicular direction of the housing.

With the indwelling needle assembly structured following the present preferred embodiment, the support mechanism realizes coaxial delivery of the delivery operating part with respect to the housing. Besides, a lock part that blocks the pressing operation of the operating member can be constituted by utilizing the positioning state of the delivery operating part by the support mechanism.

A fifth preferred embodiment of the present invention provides the indwelling needle assembly according to any of the first through fourth preferred embodiments, wherein the positioning mechanism is constituted by a structure in which the operating member and the delivery operating part are in contact with each other in the circumferential direction of the housing.

With the indwelling needle assembly structured following the present preferred embodiment, the positioning of the delivery operation part with respect to the operating member in the circumferential direction of the housing is realized by the direct contact action between the operating member and the delivery operation part.

A sixth preferred embodiment of the present invention provides the indwelling needle assembly according to any of the first through fifth preferred embodiments, wherein one of the operating member and the delivery operating part includes a positioning concave part opening toward the other of the operating member and the delivery operating part, and the other of the operating member and the delivery operating part includes a positioning convex part projecting toward the one of the operating member and the delivery operating part, and the positioning convex part is inserted in the positioning concave part, and the positioning convex part is configured to be detached from the positioning concave part through delivery of the outer needle by means of the delivery operating part so as to constitute the lock part and the positioning mechanism.

According to the indwelling needle assembly following the present preferred embodiment, the lock part for blocking the pressing operation of the operating member and the circumferential positioning mechanism of the delivery operating part with respect to the operating member can be constituted by concave and convex mating between the positioning concave part and the positioning convex part that are detached due to delivery of the outer needle.

A seventh preferred embodiment of the present invention provides the indwelling needle assembly according to any of the first through sixth preferred embodiments, wherein the delivery operating part includes a projecting delivery piece configured to directly receive the operating force that delivers the outer needle from the housing to the distal end side, the projecting delivery piece projecting on an outer circumference of the delivery operating part at a position corresponding to the operating member of the housing in the circumferential direction.

The indwelling needle assembly structured following the present preferred embodiment facilitates using both the operating member and the projecting delivery piece by orienting them toward a position that is, for example, on the opposite side to the body surface of the patient and easy to operate at the time of sticking, or the like.

An eighth preferred embodiment of the present invention provides the indwelling needle assembly according to any of the first through seventh preferred embodiments, wherein an inner needle hub provided on a proximal end side of the inner needle is urged to the proximal end side by the urging member and arranged within the housing, an engaging part is provided to the operating member on a distal end side of the direction of pressing, the engaging part projecting into the housing and engaging with the inner needle hub so as to block movement of the inner needle hub due to the urging member, and through the movement of the operating member in the direction of pressing, the engaging part is configured to move outward from the housing so as to release engagement with the inner needle hub.

With the indwelling needle assembly structured following the present preferred embodiment, the operating member is released from the direct engaged state with the inner needle hub due to the pressing operation of the operating member, whereby the inner needle can be switched from the projecting state to the housed state in the housing by the urging means.

A ninth preferred embodiment of the present invention provides an indwelling needle assembly including: a housing; an outer needle arranged so as to project to a distal end side of the housing; and an inner needle inserted through the outer needle, the inner needle being configured to be housed in the housing by means of an urging member through pressing operation of an operating member that is provided so as to be exposed on an outer circumferential surface of the housing, wherein an inner needle hub is provided on a proximal end side of the inner needle, a cap is arranged on the proximal end side of the housing, and a moving end of the inner needle to the proximal end side by means of the urging member when the inner needle is housed in the housing is defined by the inner needle hub coming into contact with the cap, while at least one of following structures (i)-(x) is adopted as a reducing mechanism of a striking noise of the inner needle hub against the cap:
(i) a structure adopting the cap comprising an elastic material exhibiting a shock absorbing action due to elastic deformation or viscoelastic deformation during contact of the inner needle hub;
(ii) a structure in which a cushioning material that exhibits a shock absorbing action is arranged on a contact part between the inner needle hub and the cap;
(iii) a structure in which the cap, with which the inner needle hub comes into contact, is attached to a proximal end opening part of a housing body in a non-adhesive way with a protrusion for press-fit mating so as to reduce transmission of the striking noise of the inner needle hub against the cap to the housing body;
(iv) a structure in which a tubular-shaped inner circumferential surface of the cap, with which an outer circumferential portion on the proximal end side of the inner needle hub comes into contact, comprises an inclined surface that is constricted in diameter backward so as to gradually increase a contact force of the inner needle hub against the cap;
(v) a structure in which a contact site between the inner needle hub and the cap has a substantially linear shape extending in the circumferential direction and a width dimension of a contact surface is reduced so as to avoid a large striking noise due to striking by a flat surface;
(vi) a structure in which the inner needle hub and the cap are configured to come into contact with each other by contact portions extending in the circumferential direction, and at least one of the contact portions of the inner needle hub and the cap includes a notch or a recess provided partially in the circumferential direction and the contact site between the inner needle hub and the cap is made partial in the circumferential direction so as to exhibit a cushioning action due to deformation of the contact site and/or a striking noise reducing action due to decrease in a contact area;
(vii) a structure in which a rigidity of a proximal end portion of the inner needle hub that comes into contact with the cap is reduced by an inner circumferential surface that gradually expands toward a side of the cap and/or an outer circumferential surface that gradually tapers toward the side of the cap so as to exhibit a cushioning action due to striking deformation of the contact portion of the inner needle hub against the cap;
(viii) a structure in which a tube-shaped projecting part is provided to the cap with which the inner needle hub comes into contact, and the tube-shaped projecting part is fitted in the proximal end opening part of the housing body while a proximal side end of the inner needle hub is configured to be inserted into the tube-shaped projecting part so as to avoid a direct contact of the inner needle hub with the housing body during striking of the inner needle hub against the cap and to prevent generation of a noise caused by the contact with the housing body which is rigid;
(ix) a structure in which a coil spring is adopted as the urging member, and a natural length of the coil spring is set smaller than a movement distance of the inner needle hub in an axial direction so as to exhibit a striking noise reducing action due to a tensile force of the coil spring during striking between the inner needle hub and the cap; and
(x) a structure in which a hemostasis valve is attached to an outer needle hub provided on the proximal end side of the outer needle, the inner needle is arranged through the hemostasis valve, and a compression force is exerted on an outer circumferential surface of the hemostasis valve such that the hemostasis valve applies an urging force to the inner needle due to the compression force.

### EFFECT OF THE INVENTION

With the indwelling needle assembly structured following the first through eighth preferred embodiments of the present invention, an unintended housing operation of the inner needle into the housing by the operating member before the delivery operation of the outer needle is prevented. In addition, an accidental rotation of the delivery operating part can be prevented, thereby improving workability of the delivery operation of the delivery operating part, for example. Furthermore, according to the ninth preferred embodiment of the present invention, it is possible to suppress the operating noise when the inner needle is housed into the housing after sticking. It is also preferable to adopt the ninth preferred embodiment in combination with the first through eighth preferred embodiments whose assumed basic structure is roughly the same.

### BRIEF DESCRIPTION OF THE DRAWINGS

FIG. 1 is a perspective view of an indwelling needle assembly according to a first practical embodiment of the present invention shown in an initial state.
FIG. 2 is a vertical cross-sectional view of the indwelling needle assembly shown in FIG 1.
FIG. 3 is an enlarged vertical cross-sectional view of a principal part shown in FIG. 2.
FIG. 4 is a perspective view of an operating button constituting the indwelling needle assembly shown in FIG. 1 in an isolated state.
FIG. 5 is a vertical cross-sectional perspective view of the operating button shown in FIG. 4.
FIG. 6 is a perspective view of the indwelling needle assembly shown in FIG. 1 showing a state where an outer needle hub is pushed forward in a direction of sticking after sticking.
FIG. 7 is an enlarged vertical cross-sectional view of a principal part of the indwelling needle assembly shown in FIG. 1 showing a state where the operating button is pushed down, and showing a state before an inner needle moves into a housing.
FIG. 8 is a vertical cross-sectional view of the indwelling needle assembly shown in FIG. 1 showing a state where the operating button is pushed down and the inner needle is housed in the housing.
FIG. 9 is a perspective view of an indwelling needle assembly according to a second practical embodiment of the present invention shown in an initial state.
FIG. 10 is an enlarged vertical sectional view of a principal part of the indwelling needle assembly shown in FIG. 9.
FIG. 11 is a perspective view of an operating button constituting the indwelling needle assembly shown in FIG. 9 in an isolated state.
FIG. 12 is a vertical cross-sectional perspective view of the operating button shown in FIG. 11.
FIG. 13 is a perspective view of the indwelling needle assembly shown in FIG. 9 showing a state where a slider is pushed forward in a direction of sticking after sticking.
FIG. 14 is a vertical cross-sectional view of the indwelling needle assembly shown in FIG. 9 showing a state where the operating button is pushed down and an inner needle is housed in a housing.

### EMBODIMENTS FOR CARRYING OUT THE INVENTION

Hereinafter, in order to clarify the present invention more specifically, practical embodiments of the present invention will be described in detail with reference to the drawings.

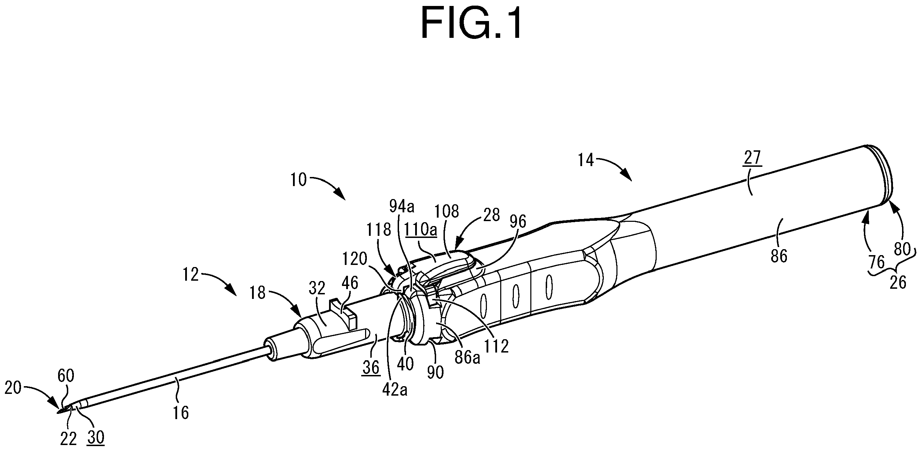

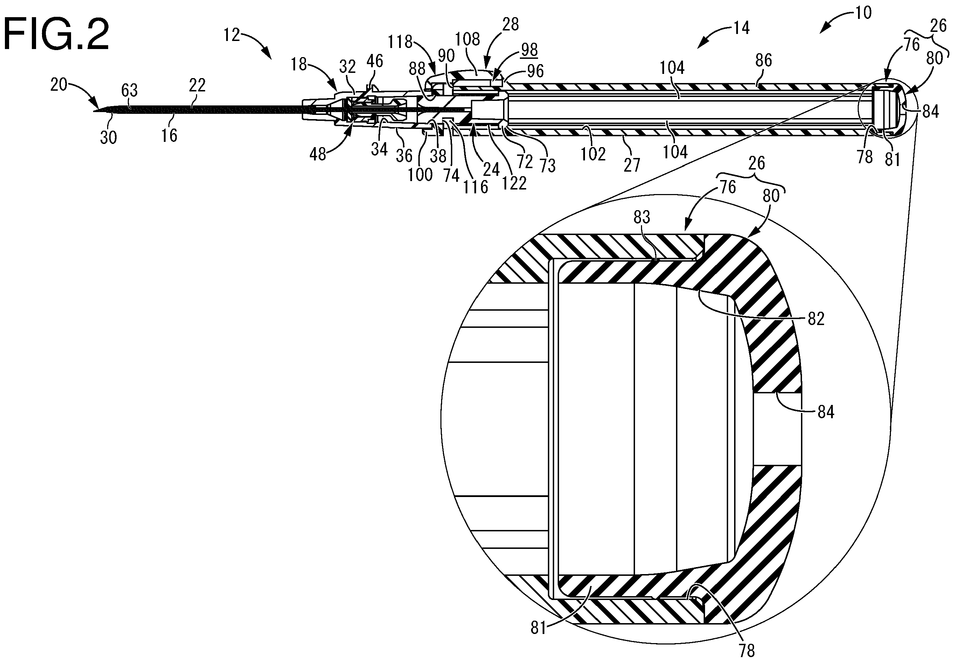

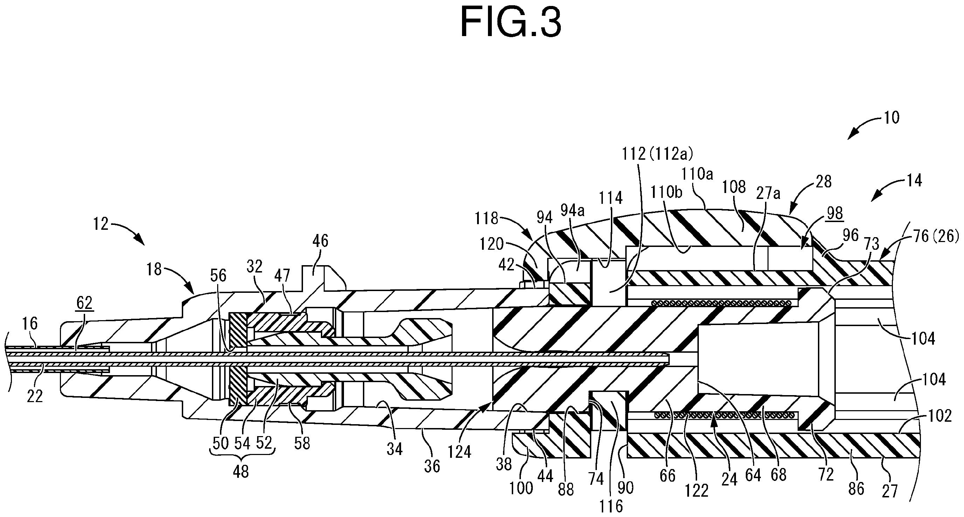

First, FIGS. 1 to 3 show an indwelling needle assembly 10 according to a first practical embodiment of the present invention shown in an initial state (a state before sticking). The indwelling needle assembly 10 includes an outer needle unit 12 and an inner needle unit 14. That is, the outer needle unit 12 includes an outer needle 16 and an outer needle hub 18 serving as a delivery operating part provided on the proximal end side of the outer needle 16. On the other hand, the inner needle unit 14 includes an inner needle 22 having a needle tip 20 at its distal end, an inner needle hub 24 provided on the proximal end side of the inner needle 22, and a housing 26 that supports the inner needle 22 and the inner needle hub 24. The indwelling needle assembly 10 is constituted by the inner needle 22 of the inner needle unit 14 being insert through the outer needle unit 12 from the proximal end side. Then, after sticking of the indwelling needle assembly 10, through pressing operation of an operating button 28 serving as an operating member that is provided so as to be exposed on an outer circumferential surface 27 of the housing 26, the inner needle 22 is configured to be pulled out of the outer needle 16 while moving into the housing to be housed therein, and the outer needle unit 12 is configured to be indwelled in the skin of the patient. In the following description, the axial direction refers to the left-right direction in FIG. 2, which is the needle axis direction of the outer needle 16 and the inner needle 22. Besides, the distal end side refers to the left side in FIG. 2 which is the needle tip 20 side of the inner needle 22, while the proximal end side refers to the right side in FIG. 2 which is the side operated by the user. Further, the upward direction and the downward direction refer to the upward direction and the downward direction in FIG. 2, respectively.

Described more specifically, in the outer needle unit 12, the outer needle 16 has a tube shape and is made of a material having appropriate flexibility, for example, various types of soft resins such as ethylene-tetrafluoroethylene copolymer (ETFE), polyurethane, and polyether nylon resin. The outer circumferential surface of the distal end portion of the outer needle 16 comprises a tapered outer circumferential surface 30, which can reduce the puncture resistance to the living body. Besides, by providing a through hole in the peripheral wall of the distal end portion of the outer needle 16, it is also possible to improve the flow efficiency of the fluid with respect to the outer needle 16, or the like.

Moreover, the outer needle hub 18 provided on the proximal end side of the outer needle 16 is made of a rigid synthetic resin material such as polypropylene, and has a generally round tubular shape including a peripheral wall 32 extending in the axial direction overall. The peripheral wall 32 of the present practical embodiment has a stepped, generally round tubular shape, and its distal end side has a smaller diameter in both the inside diameter dimension and the outside diameter dimension than its proximal end side. The proximal end of the outer needle 16 is inserted into the distal end portion having a small diameter, and a process such as bonding is performed as necessary, so that the proximal end of the outer needle 16 is fixedly supported by the outer needle hub 18. On an inner circumferential surface 34 and an outer circumferential surface 36 of the outer needle hub 18, the proximal side portions with a large diameter each have a tapered surface shape whose diameter gradually increases toward the proximal end side overall.

Further, a proximal end opening part 38 on the outer circumferential surface 36 of the outer needle hub 18 is provided with a roughly annular flange part 40 projecting to the radially outer side, and a male thread is formed on the outer circumferential surface of the flange part 40. By forming such a male thread, after the outer needle unit 12 is indwelled in the skin of the patient, for example, a luer-lock type syringe or the like can be connected to the proximal end opening part 38 of the outer needle hub 18.

The flange part 40 is provided with a lock groove 42 serving as a positioning concave part extending in the axial direction in a part of the circumference (the upper side in FIG. 2). The lock groove 42 opens to the radially outer side (upward) and is formed across the entire length of the flange part 40 in the thickness direction (the axial direction), while opening to the distal side end face and the proximal side end face of the flange part 40. Besides, the groove depth dimension (the dimension in the radial direction) of the lock groove 42 is roughly equal to the projecting dimension of the flange part 40 from the outer circumferential surface 36 of the outer needle hub 18. That is, the flange part 40 is divided in the circumferential direction by the lock groove 42, and circumferentially opposite portions 42a, 42a of the flange part 40 sandwiching the lock groove 42 are opposed to each other with a predetermined distance therebetween.

Meanwhile, in the flange part 40, a positioning concave groove 44 extending in the axial direction is provided on the diametrically opposite side to the lock groove 42. The positioning concave groove 44 also opens to the radially outer side (downward) and is formed across the entire length of the flange part 40 in the thickness direction (the axial direction), while opening to the distal side end face and the proximal side end face of the flange part 40. Moreover, the groove depth dimension (the dimension in the radial direction) of the positioning concave groove 44 is roughly equal to the projecting dimension of the flange part 40 from the outer circumferential surface 36 of the outer needle hub 18, that is, the flange part 40 is also divided in the circumferential direction by the positioning concave groove 44.

Besides, on the outer circumferential surface 36 of the outer needle hub 18, an operating piece 46 serving as a projecting delivery piece and projecting to the radially outer side (upward) is provided on a part of the circumference (the upper side in FIG. 2). In the present practical embodiment, the operating piece 46 has a generally rectangular plate shape having a predetermined width dimension. After sticking of the indwelling needle assembly 10, the user puts a finger on the operating piece 46 and delivers the outer needle hub 18 forward in the direction of sticking (toward the distal end side). That is, during the delivery operation of the outer needle hub 18 to the distal end side, the operating piece 46 is configured to directly receive the operating force for delivering the outer needle hub 18 to the distal end side.

Moreover, a positioning projection 47 for a hemostasis valve mechanism projecting to the radially inner side is provided in the axially middle portion on the inner circumferential surface 34 of the outer needle hub 18. In the present practical embodiment, the four positioning projections 47, 47, 47, 47 are formed at roughly equal intervals on the circumference.

Furthermore, a hemostasis valve mechanism 48 is housed inside the outer needle hub 18. Whereas the structure of the hemostasis valve mechanism 48 is not limited in any way, in the present practical embodiment, the hemostasis valve mechanism 48 comprises a hemostasis valve 50, a pusher 52, and a pusher guide 54.

The hemostasis valve 50 is a disc valve having a slit 56 formed in the center, and is formed of a rubber elastic body or an elastomer having elasticity. The shape of the slit 56 may be a straight-line shape, a cross shape, or the like, and is not limited in any way. However, in the present practical embodiment, the slit 56 has a radial shape extending in three directions from the center at roughly equal intervals in the circumferential direction (at about 120-degree intervals).

Additionally, the pusher 52 has a roughly tubular shape overall, and the inside diameter dimension is larger than the outside diameter dimension of the inner needle 22. Moreover, the pusher guide 54 has a roughly tubular shape overall, and the inside diameter dimension is roughly equal to or slightly larger than the maximum outside diameter dimension at the distal end portion of the pusher 52, while the outside diameter dimension is roughly equal to the inside diameter dimension of the outer needle hub 18. An annular concave groove 58 that opens to the radially outer side is formed on the outer circumferential surface of the pusher guide 54.

The outer diameter dimension of the hemostasis valve 50, in the isolated state before being attached to the outer needle hub 18, is slightly larger than the inside diameter dimension of the peripheral wall 32 of the outer needle hub 18. With this configuration, when the hemostasis valve 50 is attached to the outer needle hub 18, the hemostasis valve 50 is compressed in the radial direction (for example, the vertical direction in FIG. 2), and is housed inside the outer needle hub 18 with the slit 56 closed. Besides, the pusher 52 and the pusher guide 54 are located on the proximal end side of the hemostasis valve 50 such that the pusher 52 is inserted in the pusher guide 54. That is, the pusher guide 54 is arranged radially between the pusher 52 and the outer needle hub 18, and the positioning projection 47 of the outer needle hub 18 and the annular concave groove 58 of the pusher guide 54 are mutually mated in a concave and convex manner, so that the pusher guide 54 is attached to the outer needle hub 18. Further, the outer circumferential surface of the distal end portion of the pusher 52 is roughly in contact with the inner circumferential surface of the pusher guide 54, making it possible for the pusher 52 to slide and move in the length direction (the axial direction) with respect to the pusher guide 54. Moreover, in the present practical embodiment, in the initial state, the distal end surfaces of the pusher 52 and the pusher guide 54 are located in contact with the proximal end surface of the hemostasis valve 50.

In the initial state shown in FIG. 1 and the like, the inner needle 22 is inserted through the hemostasis valve 50. Meanwhile, as will be described later, after sticking of the indwelling needle assembly 10, the inner needle 22 is moved to the proximal end side, so that the slit 56 of the hemostasis valve 50 is closed off. Besides, after the outer needle unit 12 is indwelled in the skin of the patient, a male luer such as a syringe is inserted from the proximal end opening part 38 of the outer needle hub 18, so that the distal end of the male luer pushes the pusher 52 in the distal end direction, and the pusher 52 is guided by the pusher guide 54 to move to the distal end side, thereby opening the slit 56 of the hemostasis valve 50. By so doing, the inside of the outer needle 16, the inside of the outer needle hub 18 (the inside of the pusher 52), and the inside of the syringe or the like communicate with one another, thereby injecting, for example, the drug solution or the like in the syringe into the blood vessel of the patient through the outer needle unit 12. By removing the syringe or the like from the outer needle unit 12 after injecting the drug solution or when the injection is interrupted, the pusher 52 is pushed back to the initial position by the elastic recovering action of the hemostasis valve 50, and the slit 56 is rapidly closed.

On the other hand, the inner needle 22 in the inner needle unit 14 is a hollow needle having the sharply shaped needle tip 20, and is formed of a known material such as stainless steel, aluminum, titanium, and an alloy thereof, for example. That is, the needle tip 20 of the inner needle 22 is provided with a blade surface 60 that spreads in a direction inclining with respect to the axial direction, so that the inner needle 22 can be easily stuck into the skin of the patient or the like. In the present practical embodiment, the blade surface 60 is oriented upward. The inner needle 22 may be a solid needle.

Further, the outside diameter dimension of the inner needle 22 is smaller than the inside diameter dimension of the outer needle 16, and with the inner needle 22 inserted through the outer needle 16, an annular gap 62 is formed radially between the inner needle 22 and the outer needle 16. That is, the gap 62 formed radially between the inner needle 22 and the outer needle 16 communicates with the internal space of the outer needle hub 18. Furthermore, the distal end portion of the inner needle 22 is provided with a through hole 63 that penetrates the peripheral wall of the inner needle 22. At the time of sticking the indwelling needle assembly 10, the blood that has flowed into the inner needle 22 flows backward through the through hole 63 and the gap 62 to the portion closed by the hemostasis valve 50 in the outer needle hub 18. Therefore, by forming the outer needle hub 18 with a transparent material, blood return (flashback) can be easily confirmed at the time of sticking the indwelling needle assembly 10.

Moreover, the inner needle hub 24 provided on the proximal end side of the inner needle 22 is made of a rigid synthetic resin material such as polypropylene, and has an elongated, generally round tubular shape overall. An annular stepped surface 64 is formed on the inner circumferential surface of the inner needle hub 24, and the distal end side of the stepped surface 64 comprises a small-diameter part 66 having a smaller inside diameter dimension, while the proximal end side of the stepped surface 64 comprises a large-diameter part 68 having an inside diameter dimension larger than that of the small-diameter part 66. The inside diameter dimension of the small-diameter part 66 is roughly equal to or slightly larger than the outside diameter dimension of the inner needle 22. The proximal end of the inner needle 22 is inserted into the small-diameter part 66, and is subjected to a treatment such as bonding if necessary, whereby the proximal end of the inner needle 22 is fastened to the small-diameter part 66. By so doing, the proximal end of the inner needle 22 is fixedly supported by the inner needle hub 24.

Besides, the outside diameter dimensions of the small-diameter part 66 and the large-diameter part 68 are roughly equal to each other, and the outer circumferential surface of the small-diameter part 66 and the outer circumferential surface of the large-diameter part 68 are continuous with each other in the axial direction with no step. In the present practical embodiment, the distal end portion of the small-diameter part 66 has a tapered shape roughly corresponding to the proximal end opening part 38 of the outer needle hub 18. As will be described later, when the distal end portion of the small-diameter part 66 (the insertion part 124) is inserted into the inside of the outer needle hub 18 from the proximal end opening part 38 of the outer needle hub 18, the inner circumferential surface 34 of the outer needle hub 18 and the outer circumferential surface of the distal end portion of the small-diameter part 66 (the insertion part 124) are in contact with each other with almost no gap. However, the outside diameter dimension at the distal end portion of the small-diameter part 66 may be smaller than the inside diameter dimension at the proximal end opening part 38 of the outer needle hub 18, and a gap may be formed between the inner circumferential surface of the outer needle hub 18 and the outer circumferential surface of the distal end portion of the small-diameter part 66 (the insertion part 124).

For example, the proximal end opening part of the inner needle hub 24 (namely, the proximal end opening part of the large-diameter part 68) is provided with a filter that does not allow liquid to pass through but allows gas to pass through. With this configuration, at the time of sticking the indwelling needle assembly 10, the blood that has flowed in through the inner hole of the inner needle 22 is stored inside the large-diameter part 68, while the blood can be prevented from leaking from the proximal end opening part of the inner needle hub 24. It would also be acceptable to form the inner needle hub 24 and the housing 26 with a transparent material so that blood return (flashback) is confirmed by the blood stored inside the large-diameter part 68 at the time of sticking the indwelling needle assembly 10.

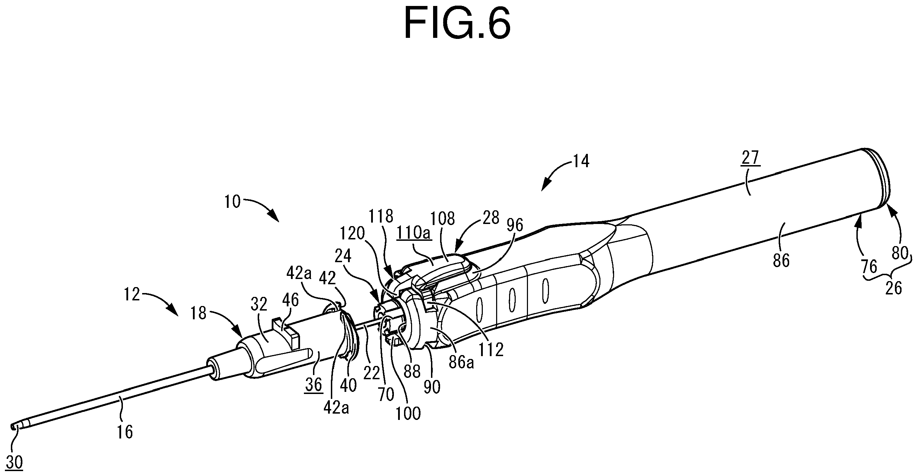

Furthermore, the outer circumferential surface of the inner needle hub 24 is provided with a guide concave groove 70 (see FIG. 6 and the like) that extends across the entire length in the axial direction and opens to the radially outer side. In the present practical embodiment, the guide concave groove 70 has a generally rectangular cross-sectional shape, and four guide concave grooves 70, 70, 70, 70 are formed on the outer circumferential surface of the inner needle hub 24 at roughly equal intervals on the circumference.

Moreover, on the outer circumferential surface of the inner needle hub 24, a roughly annular flange-shaped part 72 projecting to the radially outer side is provided to the proximal end opening part (namely, the proximal end opening part of the large-diameter part 68). On the outer circumferential surface of the flange-shaped part 72, concave grooves (not shown) extending in the axial direction and opening to the radially outer side are provided at positions corresponding to the aforementioned four guide concave grooves 70, 70, 70, 70. In other words, the guide concave grooves 70, 70, 70, 70 provided on the outer circumferential surface of the inner needle hub 24 are formed as far as the flange-shaped part 72, and the flange-shaped part 72 has a shape that is divided in the circumferential direction. In the present practical embodiment, at the proximal end on the outer circumferential surface of the flange-shaped part 72, a tapered surface 73 that gradually decreases in diameter toward the proximal end side is provided. Further, the inner circumferential surface of the proximal end opening part of the inner needle hub 24 is also provided with a taper that expands toward the proximal end side, so that the proximal end of the inner needle hub 24 is made tapered and thin, thereby reducing the rigidity.

Furthermore, on the outer circumferential surface of the inner needle hub 24, an engaging concave part 74 that opens to the radially outer side is formed in the axially middle portion (namely, the axially middle portion of the small-diameter part 66). The engaging concave part 74 is formed in a part of the circumference (the lower side in FIG. 2), and opens downward in the present practical embodiment.

The inner needle hub 24 having such a structure is supported by the housing 26. That is, the housing 26 has a roughly bottomed tubular shape overall, and the inner needle hub 24 is arranged inside the housing 26. The housing 26 is made of a rigid synthetic resin similar to that of the outer needle hub 18 and the inner needle hub 24. Besides, the housing 26 includes a housing body 76 having a roughly tubular shape and a cap 80 that closes off a proximal end opening part 78 of the housing body 76.

The cap 80 is provided with an annular projecting part 81 projecting to the distal end side from a main body having a generally circular disk shape overall, and is integrally formed of an elastic body such as rubber and an elastomer. In the present practical embodiment, the inner circumferential surface of the projecting part 81 has an inclined surface 82 that increases in diameter toward the distal end side. The projecting part 81 is fitted into the proximal end opening part 78 of the housing body 76, and the outer peripheral end of the main body and the outer circumferential surface of the projecting part 81 are welded or bonded to the axial end surface and the inner circumferential surface of the housing body 76 if necessary, so that the cap 80 is fastened to the proximal end opening part 78 of the housing body 76. The cap 80 need not be fixed to the proximal end opening part 78 of the housing body 76. For example, it would also be acceptable to provide the outer circumferential surface of the projecting part 81 with a protrusion 83 having a tapered cross section such as a roughly semicircular shape so as to extend about the entire circumference in the circumferential direction or partially in the circumferential direction. By the protrusion 83 being press-fitted into the proximal end opening part 78 of the housing body 76, the cap 80 can be fixed by being press-fitted in a non-adhesive way. Such a protrusion for press-fit mating may be provided on the inner circumferential surface of the proximal end opening part 78 of the housing body 76.

Additionally, a through hole 84 penetrates the center portion of the cap 80 in the thickness direction, and as will be described later, when the inner needle hub 24 moves inside the housing 26, the air inside the housing 26 will be discharged to the outside through the through hole 84. With this configuration, the risk that the movement of the inner needle hub 24 is hindered by the action of the air spring due to the air inside the housing 26 can be reduced.

Moreover, the housing body 76 of the present practical embodiment includes a tubular peripheral wall 86 having an axial dimension larger than an axial dimension from the needle tip 20 of the inner needle 22 to the proximal end of the inner needle hub 24. The inside diameter dimension of the peripheral wall 86 is roughly constant across approximately the entire length in the axial direction, and is slightly larger than the outside diameter dimension of the flange-shaped part 72 at the proximal end of the inner needle hub 24. In a distal end opening part 88 of the housing body 76, the inside diameter dimension of the peripheral wall 86 is smaller than those of the other portions, and is smaller than the outside diameter dimension of the flange-shaped part 72 of the inner needle hub 24 while being roughly equal to or slightly larger than the outside diameter dimensions of the portions other than the flange-shaped part 72 (namely, the small-diameter part 66 and the large-diameter part 68). With this configuration, the inner needle hub 24 arranged in the housing 26 is movable within the housing 26 in the axial direction, and can be prevented from falling out of the housing 26 through the distal end opening part 88. In the present practical embodiment, the inside diameter dimension of the distal end opening part 88 of the housing body 76 is roughly equal to the inside diameter dimension of the proximal end opening part 38 of the outer needle hub 18.

An insertion hole 90 penetrates the distal end portion of the peripheral wall 86 in the vertical direction. The insertion hole 90 has a generally rectangular shape in plan view, and its widthwise dimension (the dimension in the direction perpendicular to the plane of the page in FIG. 2) is larger than its axial dimension. The widthwise dimension of the insertion hole 90 is smaller than the widthwise dimension (the outside diameter dimension) of the peripheral wall 86, and the insertion hole 90 penetrates the upper portion and the lower portion of the tubular peripheral wall 86, so as to open to the upper side and the lower side of the outer circumferential surface 27 of the housing 26.

Besides, on the outer circumferential surface 27 of the distal end opening part 88 of the housing 26 (the housing body 76), a mating groove 94 extending in the axial direction is formed in a part of the circumference (the upper side in FIG. 2). The mating groove 94 is provided on the distal end side with respect to the insertion hole 90 on the upper side of the housing body 76, and communicates with the insertion hole 90. The groove width dimension of the mating groove 94 (the dimension in the direction perpendicular to the plane of the page in FIG. 2) is roughly equal to the groove width dimension of the lock groove 42 provided to the flange part 40 of the outer needle hub 18. That is, the circumferentially (widthwise) opposite portions 94a, 94a of the peripheral wall 86 sandwiching the mating groove 94 are opposed to each other with a predetermined distance therebetween.

Furthermore, on the outer circumferential surface 27 of the housing 26 (the housing body 76), a surrounding projection 96 projecting to the radially outer side (upward) is formed on a part of the circumference (the upper side in FIG. 2). The surrounding projection 96 extends in a roughly U shape or a roughly square-bracket shape with a roughly triangular vertical cross section, and opens to the distal end side. Also, the surrounding projection 96 is provided on the proximal end side with respect to the insertion hole 90, and the distal end opening part of the surrounding projection 96 communicates with the insertion hole 90. That is, the area surrounded by the surrounding projection 96 is defined as an arrangement area 98 that is concave opening upward with respect to the surrounding projection 96. In the present practical embodiment, the bottom surface of the arrangement area 98, namely, a portion 27a surrounded by the surrounding projection 96 on the outer circumferential surface 27 of the housing 26 is located on the radially inner side than the other portions on the outer circumferential surface 27 so as to obtain a large vertical dimension of the arrangement area 98. Further, by providing the portion 27a surrounded by the surrounding projection 96 on the radially inner side than the other portions of the outer circumferential surface 27, it is possible to make the projection height of the surrounding projection 96 relatively high. By so doing, the guiding action of the surrounding projection 96 in the vertical direction at the time of the pressing operation of the operating button 28 can be more effectively exhibited.

Furthermore, in the distal end opening part 88 of the housing body 76, a positioning protrusion 100 projecting to the distal end side is formed on the diametrically opposite side to the mating groove 94. The positioning protrusion 100 has a generally rectangular shape in plan view, and the widthwise dimension of the positioning protrusion 100 is roughly equal to or slightly smaller than the widthwise dimension of the positioning concave groove 44 provided to the flange part 40 of the outer needle hub 18.

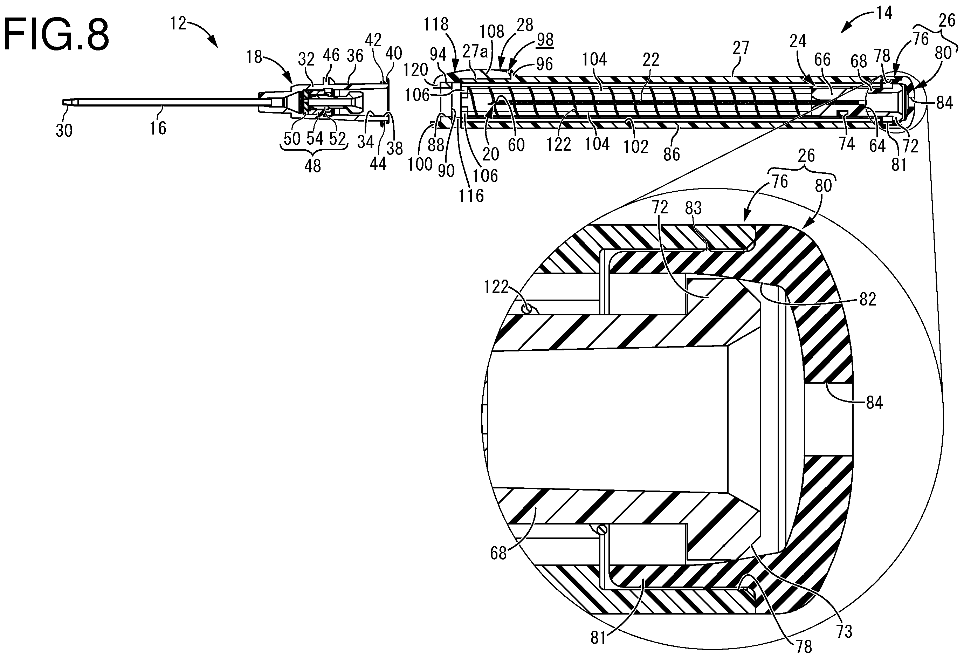

On the other hand, an inner circumferential surface 102 of the housing body 76 is provided with a guide protrusion 104 projecting from the peripheral wall 86 to the radially inner side. The guide protrusion 104 has a generally rectangular cross-sectional shape, and is formed across the roughly entire length in the axial direction on the proximal end side with respect to the insertion hole 90 in the peripheral wall 86. In the present practical embodiment, the guide protrusion 104 is formed at four locations on the circumference at the circumferential positions corresponding to the concave grooves (the guide concave grooves 70) provided on the outer circumferential surface of the flange-shaped part 72 of the inner needle hub 24. Further, at the distal ends of the guide protrusions 104, 104, 104, 104, namely, at the portions adjacent to the insertion hole 90 on the proximal end side, the inner projections 106, 106, 106, 106 whose projecting dimension to the radially inner side is made large (see FIG. 8) are formed. The number of the guide protrusions 104, the concave grooves (the guide concave grooves 70), and the inner projections 106 is not limited, but may be, for example, three, or five or more.

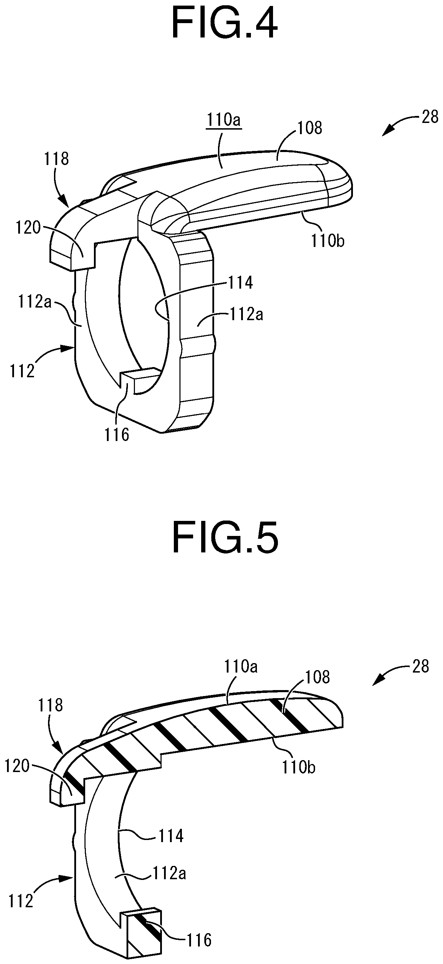

The operating button 28 as shown in FIGS. 4 and 5 is attached to the housing 26 having the structure as described above. The operating button 28 is formed of a rigid synthetic resin similar to that of the housing 26, and includes a base part 108 having a similar shape to the area surrounded by the surrounding projection 96 (the arrangement area 98) in plan view. An upper surface 110a of the base part 108 comprises a curved surface that is convex upward, while a lower surface 110b thereof comprises a flat surface that extends in the axial direction. In this way, since the upper surface 110a of the base part 108 is a curved surface, a large contact area with the fingers of the user is obtained during the pressing operation of the operating button 28, thereby improving operability of the pressing operation.

An annular part 112 projecting downward is formed at the distal end portion of the base part 108. That is, the annular part 112 has contours of a generally rectangular plate shape in which the vertical dimension is larger than the widthwise dimension viewed in the axial direction. Besides, an inner hole 114 having a roughly circular shape penetrates the center of the annular part 112 in the thickness direction (the axial direction). The upper portion of the annular part 112 is integrally formed with the base part 108. The inside diameter dimension of the annular part 112 (the diameter dimension of the inner hole 114) is roughly equal to the inside diameter dimension of the peripheral wall 86 of the housing body 76 (the inside diameter dimension of the portion in the distal end opening part 88 other than the portion where the inside diameter is reduced). The axial dimension and the widthwise dimension of the annular part 112 are roughly equal to or slightly smaller than the axial dimension and the widthwise dimension of the insertion hole 90 in the peripheral wall 86 of the housing body 76, respectively.

Further, in the annular part 112, an engaging convex part 116 serving as an engaging part is provided on the diametrically opposite side to the side where the base part 108 is provided (namely, the lower side which is the distal end side of the direction of pressing of the operating button 28) and projects to the radially inner side (upward). That is, the inside diameter dimension of the annular part 112 is reduced at the formation position of the engaging convex part 116, and the inside diameter dimension at the formation position of the engaging convex part 116, namely, the vertical distance between the base part 108 and the engaging convex part 116, is roughly equal to the inside diameter dimension of the distal end opening part 88 in the housing body 76.

Here, at the center portion of the base part 108 in the width direction (the direction perpendicular to the plane of the page in FIG. 2), a projecting lock piece 118 serving as a positioning convex part is provided so as to project to the distal end side. The distal end of the projecting lock piece 118 is defined as a contact part 120 that bends and projects to the radially inner side (downward). The widthwise dimension of the projecting lock piece 118 is roughly equal to or slightly smaller than the groove width dimensions of the mating groove 94 provided in the distal end opening part 88 of the housing body 76 and the lock groove 42 provided in the flange part 40 of the outer needle hub 18.

The inner needle unit 14 includes the inner needle 22, the inner needle hub 24, the housing 26, and the operating button 28 having the above-mentioned structure. That is, the inner needle 22 is fixed to the inner needle hub 24, the inner needle 22 and the inner needle hub 24 are inserted from the proximal end opening part 78 of the housing body 76, and the proximal end opening part 78 is closed off by the cap 80, so that the inner needle hub 24 is housed within the housing 26. When assembling the inner needle hub 24 and the housing 26, the guide protrusions 104 provided on the inner circumferential surface 102 of the housing body 76 are inserted into the concave grooves provided to the flange-shaped part 72 of the inner needle hub 24, while the inner projections 106 provided at the distal ends of the guide protrusions 104 are inserted into the guide concave grooves 70 provided on the outer circumferential surface of the inner needle hub 24. This arrangement prevents relative rotation of the inner needle 22, the inner needle hub 24, and the housing 26 in the circumferential direction, and the blade surface 60 of the inner needle 22 and the operating button 28 are aligned in the circumferential direction so as to be both oriented upward.

Additionally, the operating button 28 is attached to the housing 26. Specifically, the annular part 112 of the operating button 28 is inserted in the insertion hole 90 provided at the distal end portion of the housing body 76 from above, and the base part 108 is housed within the arrangement area 98 surrounded by the surrounding projection 96. That is, in the present practical embodiment, the operating button 28 is arranged so as to be exposed in the arrangement area 98 provided on the outer circumferential surface 27 of the housing 26 (the housing body 76), and the operating button 28 is located on the upper side of the housing 26. By arranging the bottom surface 27a of the arrangement area 98 and the lower surface 110b of the base part 108 so as to be remote from each other in the vertical direction, the operating button 28 is movable with respect to the housing 26 in the vertical direction. In the initial state, the projecting lock piece 118 projecting from the base part 108 to the distal end side is located on the radially outer side (the upper side) of the mating groove 94 provided in the distal end opening part 88 of the housing body 76.

Moreover, by the annular part 112 being inserted into the insertion hole 90 and/or the base part 108 being housed within the arrangement area 98 in this way, relative rotation of the housing 26 and the operating button 28 in the circumferential direction is prevented. That is, among the walls constituting the annular part 112, widthwise opposite walls 112a, 112a sandwiching the inner hole 114 are in contact with the widthwise opposite walls 86a, 86a sandwiching the insertion hole 90 in the peripheral wall 86 of the housing body 76, so as to prevent the relative rotation of the housing 26 and the operating button 28 in the circumferential direction. Alternatively, the base part 108 is in contact with the surrounding projection 96 to prevent the relative rotation of the housing 26 and the operating button 28 in the circumferential direction. The relative rotation prevention mechanism between the housing 26 and the operating button 28 may be constituted by only one of the annular part 112 being inserted in the insertion hole 90 and the base part 108 being housed within the arrangement area 98.

In the initial state, the inner needle 22 and the inner needle hub 24 are inserted through the inner hole 114 of the annular part 112 inserted in the insertion hole 90, and the engaging convex part 116 provided below the annular part 112 is fitted in and engaged with the engaging concave part 74 that opens onto the lower surface of the inner needle hub 24. With this configuration, the distal end portions of the inner needle 22 and the inner needle hub 24 are held in a state of projecting from the housing 26 to the distal end side. In other words, in the housing 26, the engaging convex part 116 projects to the radially inner side, and by the engaging convex part 116 and the engaging concave part 74 of the inner needle hub 24 engaging with each other, the axial movement of the inner needle hub 24 with respect to the housing 26 is blocked.

Here, a coil spring 122 serving as an urging member is arranged between the inner needle hub 24 and the housing 26. That is, the coil spring 122 is provided axially between the flange-shaped part 72 provided at the proximal end of the inner needle hub 24 and the inner projections 106, 106, 106, 106 projecting to the radially inner side at the distal end portion of the housing 26. In the initial state, the flange-shaped part 72 and the inner projections 106, 106, 106, 106 are close to each other, and the coil spring 122 is compressed by the flange-shaped part 72 and the inner projections 106. With this arrangement, the inner needle hub 24 is subjected to an urging force toward the proximal end side of the housing 26 due to an elastic recovery force of the coil spring 122. Besides, the movement of the inner needle hub 24 to the proximal end side of the housing 26 due to the urging force is blocked by the engagement between the engaging convex part 116 and the engaging concave part 74.

By attaching the outer needle unit 12 to the inner needle unit 14 having such a structure, the indwelling needle assembly 10 of the present practical embodiment is constituted. That is, the inner needle 22 is inserted from the proximal end opening part 38 of the outer needle hub 18, penetrates the hemostasis valve mechanism 48 and the outer needle 16, and the needle tip 20 of the inner needle 22 projects from the outer needle 16.

Here, in the present practical embodiment, since the hemostasis valve 50 is compressed in the radial direction by the peripheral wall 32 of the outer needle hub 18. Thus, by the inner needle 22 being inserted through the slit 56 of the hemostasis valve 50, a radial compression force of the hemostasis valve 50 is exerted on the inner needle 22. Due to the compression force of the hemostasis valve 50, the hemostasis valve 50 exerts an urging force on the inner needle 22, and the inner surface of the slit 56 is pressed against the outer circumferential surface of the inner needle 22. With this configuration, when the inner needle 22 and the inner needle hub 24 move toward the proximal end side, which will be described later, the radial compression force of the hemostasis valve 50 is exerted on the inner needle 22, and the moving speed of the inner needle 22 and the inner needle hub 24 to the proximal end side is suppressed, thereby reducing the striking noise between the inner needle hub 24 and the cap 80 when the inner needle 22 is pulled out.

Moreover, the distal end portion of the inner needle hub 24 enters the inside of the outer needle hub 18 from the proximal end opening part 38 of the outer needle hub 18. With this configuration, the distal end portion of the inner needle hub 24 comprises an insertion part 124 that enters the inside from the proximal end opening part 38 of the outer needle hub 18. In the present practical embodiment, the outside diameter dimension of the distal end portion of the inner needle hub 24 (the small-diameter part 66) is roughly equal to the inside diameter dimension of the proximal end opening part 38 of the outer needle hub 18. Thus, the outer circumferential surface of the insertion part 124 and the inner circumferential surface 34 of the outer needle hub 18 come into contact with each other with almost no gap. However, a gap may be formed between the outer circumferential surface of the insertion part 124 and the inner circumferential surface 34 of the outer needle hub 18. The distal end opening part 88 of the housing 26 (the housing body 76) is located adjacent to the proximal end opening part 38 of the outer needle hub 18 in the axial direction, in other words, the outer needle hub 18 is located on the distal end side of the housing 26.

Besides, in such an assembled state of the inner needle unit 14 and the outer needle unit 12, the projecting lock piece 118 projecting from the base part 108 to the distal end side in the operating button 28 extends as far as the radially outer side of the flange part 40 of the outer needle hub 18, and the contact part 120, which is the distal end portion of the projecting lock piece 118, is inserted in the lock groove 42 provided to the flange part 40 of the outer needle hub 18 from the radially outer side (the upper side). That is, the contact part 120 of the projecting lock piece 118 is circumferentially in contact with the circumferentially opposite portions 42a, 42a sandwiching the lock groove 42 of the flange part 40, thereby preventing relative rotation of the operating button 28 and the outer needle hub 18, namely, the inner needle unit 14 and the outer needle unit 12, in the circumferential direction. Therefore, in the present practical embodiment, one of the positioning mechanisms for positioning the outer needle hub 18 with respect to the operating button 28 in the circumferential direction includes the projecting lock piece 118 and the lock groove 42.

Furthermore, in the assembled state of the inner needle unit 14 and the outer needle unit 12, the positioning protrusion 100 provided on the diametrically opposite side to the projecting lock piece 118 in the inner needle unit 14 is inserted in the positioning concave groove 44 provided to the flange part 40 of the outer needle hub 18. That is, the relative rotation in the circumferential direction of the housing 26 and the outer needle hub 18, namely, the inner needle unit 14 and the outer needle unit 12, is prevented also by the positioning protrusion 100 being circumferentially in contact with the circumferentially opposite portions sandwiching the positioning concave groove 44 of the flange part 40. Here, since the operating button 28 is attached to the housing 26 nonrotatably in the circumferential direction, by the positioning protrusion 100 being inserted in the positioning concave groove 44, the outer needle hub 18 is positioned with respect to the operating button 28 in the circumferential direction via the housing 26. Therefore, another one of the positioning mechanisms for positioning the outer needle hub 18 with respect to the operating button 28 in the circumferential direction includes the relative rotation prevention mechanism between the operating button 28 and the housing 26, the positioning protrusion 100, and the positioning concave groove 44.

By the contact part 120 of the projecting lock piece 118 being inserted from the radially outer side (the upper side) of the lock groove 42 in this way, in the formation position of the flange part 40, the peripheral wall 32 of the outer needle hub 18 is sandwiched in the radial direction between the contact part 120 of the projecting lock piece 118 and the insertion part 124 which is the distal end portion of the inner needle hub 24. The peripheral wall 32 of the outer needle hub 18 and the contact part 120 of the projecting lock piece 118 and/or the insertion part 124 may be in contact with one another, or may be remote from one another by a predetermined distance.

Here, the contact part 120 of the projecting lock piece 118 is inserted from the radially outer side (the upper side) of the lock groove 42. Thus, by the contact part 120 being in contact with the peripheral wall 32 (the outer circumferential surface 36) of the outer needle hub 18 which is the groove bottom surface of the lock groove 42 in the axis-perpendicular direction of the housing 26, namely, in the vertical direction, movement of the operating button 28 downward, which is the direction of pressing (namely, pressing operation), is blocked. That is, in the present practical embodiment, the lock part that blocks the pressing operation of the operating button 28 is constituted by the projecting lock piece 118 being inserted from the radially outer side of the lock groove 42. Since the lock groove 42 and the projecting lock piece 118 are respectively provided to the upper side of the outer needle hub 18 and the operating button 28 located on the upper side of the housing 26, the lock part is provided so as to be exposed to the radially outer side on the upper side of the indwelling needle assembly 10, that is, at the same position on the circumference as the operating button 28. The lower end surface of the contact part 120 does not need to be in contact with the groove bottom surface of the lock groove 42 (the outer circumferential surface 36 of the outer needle hub 18). The lower end surface of the contact part 120 and the groove bottom surface of the lock groove 42 may be remote from each other to the extent that the engagement between the engaging convex part 116 and the engaging concave part 74 will not be released due to the operating button 28 moving downward.

In the indwelling needle assembly 10 in the initial state, the center axis of the outer needle hub 18 and the center axis of the housing 26 are aligned with each other, and the outer needle hub 18 is positioned and supported roughly coaxially with respect to the housing 26. In the present practical embodiment, the support mechanism that positions and supports the outer needle hub 18 roughly coaxially with respect to the housing 26 is constituted by the insertion part 124, which is the distal end portion of the inner needle hub 24, being inserted from the proximal end opening part 38 of the outer needle hub 18 so that the outer circumferential surface of the insertion part 124 and the inner circumferential surface 34 of the outer needle hub 18 are in contact with each other with almost no gap. In particular, the insertion part 124 is inserted into the proximal end opening part 38 of the outer needle hub 18 with a certain degree of mating force, thereby preventing the outer needle unit 12 from accidentally falling out of the inner needle unit 14.

The indwelling needle assembly 10 having the above-mentioned structure is stuck into a blood vessel of a patient with the inner needle 22 inserted in the outer needle 16 as shown in FIGS. 1 to 3.

Then, after confirming the flashback, a finger is placed on the operating piece 46 projecting upward from the outer circumferential surface 36 of the outer needle hub 18, and the outer needle unit 12 is delivered forward in the direction of sticking (toward the distal end side). By so doing, as shown in FIG. 6, the outer needle unit 12 is moved to the distal end side with respect to the inner needle unit 14, and the distal end of the outer needle 16 is pushed deeper into the blood vessel of the patient. That is, in the present practical embodiment, the outer needle hub 18 constitutes a delivery operating part that exerts an operating force for delivering the outer needle 16 to the distal end side with respect to the housing 26. In the present practical embodiment in particular, the outer needle hub 18 is configured to be delivered to the distal end side in a state of being coaxially positioned with respect to the housing 26 by the support mechanism.

It would also be acceptable to pull out the inner needle unit 14 backward in the direction of sticking (toward the proximal end side) while delivering the outer needle unit 12 forward in the direction of sticking. By so doing, the needle tip 20 of the inner needle 22 can be removed from the blood vessel of the patient. In such a state, the proximal end opening part 38 of the outer needle hub 18 and the distal end opening part 88 of the housing 26 (the housing body 76) are remote from each other in the axial direction, and the projecting lock piece 118 is detached from the lock groove 42.

That is, by pushing out the outer needle unit 12 to the distal end side with respect to the inner needle unit 14, the contact part 120 of the projecting lock piece 118 is detached from the lock groove 42, thereby releasing the mechanism for blocking the pressing operation of the operating button 28 due to the contact between the contact part 120 of the projecting lock piece 118 and the groove bottom surface of the lock groove 42 (the peripheral wall 32 of the outer needle hub 18). Therefore, by pushing out the outer needle unit 12 forward in the direction of sticking, the pressing operation of the operating button 28 (the movement downward which is the direction of pressing) is enabled.

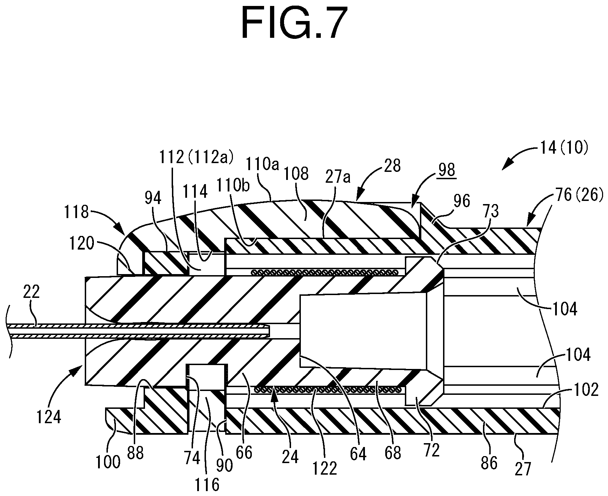

Then, as shown in FIG. 7, the finger is overlapped on the upper surface 110a of the operating button 28 and the operating button 28 is pushed downward. By so doing, the base part 108 of the operating button 28 moves downward in the arrangement area 98, and the lower surface 110b of the base part 108 and the bottom surface 27a of the arrangement area 98 are overlapped with each other, while the projecting lock piece 118 of the operating button 28 is inserted into the mating groove 94 provided in the distal end opening part 88 of the housing 26 (the housing body 76). Additionally, due to the annular part 112 of the operating button 28 moving downward, the engaging convex part 116 is detached from the engaging concave part 74, thereby releasing their engagement. That is, due to the annular part 112 moving downward, the engaging convex part 116 projecting from the inner circumferential surface 102 of the housing 26 (the housing body 76) to the radially inner side (upward) moves to the radially outer side (downward), so that the inner circumferential surface 102 of the housing 26 is formed to be roughly straight across the approximately entire length in the axial direction. As a result, the inner needle hub 24 becomes movable inside the housing 26 in the axial direction.

By so doing, as shown in FIG. 8, the inner needle hub 24 and the inner needle 22 are moved to the proximal end side within the housing 26 according to the urging force based on the elastic recovery force of the coil spring 122, and the inner needle 22 is pulled out of the outer needle 16. In the present practical embodiment, the axial dimension of the housing 26 is larger than the axial dimension from the needle tip 20 of the inner needle 22 to the proximal end of the inner needle hub 24. Thus, due to the inner needle hub 24 and the inner needle 22 moving to the proximal end side within the housing 26, the needle tip 20 of the inner needle 22 is configured to be covered and protected by the housing 26.

In the present practical embodiment, the moving end of the inner needle hub 24 toward the proximal end side is defined by the inner needle hub 24 that has moved to the proximal end side and the cap 80 provided at the proximal end of the housing 26 coming into contact with each other. Here, for example, by the synergistic action of any one or a combination of a plurality of the following structures (i) to (x), it is possible to reduce the striking noise between the inner needle hub 24 and the cap 80 when the inner needle 22 is pulled out by the pressing operation of the operating button 28 after sticking or puncture:
(i) a structure adopting the cap 80 comprising an elastic material exhibiting a shock absorbing action due to elastic deformation or viscoelastic deformation during contact of the inner needle hub 24 (note that the cap 80 has a degree of freedom in selecting the material that is greater than that of the inner needle hub 24 or the housing body 76. Moreover, since the elastic cap 80 that exhibits a shock absorbing action is mounted on the housing 26 side, the cap 80 can also effectively act as a vibration damping material that suppresses the amplification of the striking noise due to the resonance of the housing 26);
(ii) a structure in which a cushioning material that exhibits a shock absorbing action is arranged on a contact part between the inner needle hub 24 and the cap 80;
(iii) a structure in which the cap 80, with which the inner needle hub 24 comes into contact, is attached to the proximal end opening part 78 of the housing body 76 in a non-adhesive way with the protrusion 83 for press-fit mating so as to reduce transmission of the striking noise of the inner needle hub 24 against the cap 80 to the housing body 76, thereby reducing or avoiding the amplification of the striking noise due to the resonance action of the housing body 76 or the like;
(iv) a structure in which the inner circumferential surface of the projecting part 81 of the cap 80, with which the inner needle hub 24 comes into contact, comprises the inclined surface 82 that is constricted in diameter backward so as to gradually increase the contact force of the inner needle hub 24 against the cap 80;
(v) a structure in which a contact site between the inner needle hub 24 and the cap 80 has a substantially linear shape extending in the circumferential direction and the width dimension of the contact surface is reduced so as to avoid a large striking noise due to striking by a flat surface;
(vi) a structure in which at least one of the contact portions of the inner needle hub 24 and the cap 80 includes a notch or a recess provided partially in the circumferential direction and the contact site between the inner needle hub 24 and the cap 80 is made partial in the circumferential direction so as to exhibit a cushioning action due to deformation of the contact site and/or a striking noise reducing action due to decrease in the contact area;
(vii) a structure in which the rigidity of the proximal end portion of the inner needle hub 24 that comes into contact with the cap 80 is reduced by the inner circumferential surface that gradually expands toward the side of the cap 80 and/or the outer circumferential surface that gradually tapers toward the side of the cap 80 so as to exhibit a cushioning action due to striking deformation of the contact portion of the inner needle hub 24 against the cap 80;
(viii) a structure in which the tube-shaped projecting part 81 is provided to the cap 80 with which the inner needle hub 24 comes into contact, and the tube-shaped projecting part 81 is fitted in the proximal end opening part 78 of the housing body 76 while the proximal side end of the inner needle hub 24 is configured to be inserted into the tube-shaped projecting part 81 so as to avoid a direct contact of the inner needle hub 24 with the housing body 76 during striking of the inner needle hub 24 against the cap 80 and to prevent generation of a noise caused by the contact of the inner needle hub 24 with the housing body 76 which is rigid;
(ix) a structure in which the natural length of the coil spring 122 is set smaller than the movement distance of the inner needle hub 24 in the axial direction so as to exhibit a striking noise reducing action due to the tensile force of the coil spring 122 during striking between the inner needle hub 24 and the cap 80; and
(x) a structure in which the hemostasis valve 50 is attached to the outer needle hub 18 provided on the proximal end side of the outer needle 16, the inner needle 22 is arranged through the hemostasis valve 50, and the compression force is exerted on the outer circumferential surface of the hemostasis valve 50 such that the hemostasis valve 50 applies the urging force to the inner needle 22 due to the compression force.

The pressing operation of the operating button 28 (the state in FIG. 7) and the movement of the inner needle hub 24 to the proximal end side (the state in FIG. 8) occur roughly at the same time, but in the aforementioned description, these states are described separately. Also, it is preferable that the operation of pushing down the operating button 28 and housing the inner needle 22 in the housing 26 in this way is performed at the time when the needle tip 20 of the inner needle 22 is pulled out of the blood vessel of the patient and is located in the middle portion in the length direction of the outer needle 16. However, for example, the said operation may be performed with the inner needle 22 stuck in the blood vessel of the patient, or may be performed after the inner needle 22 is pulled out of the outer needle unit 12.

Then, by removing the inner needle 22 from the outer needle unit 12 in this way, the outer needle unit 12 is configured to be indwelled in the skin of the patient. By connecting an external flow path such as a syringe to the outer needle hub 18 in such a state from the proximal end opening part 38, the slit 56 of the hemostasis valve 50 is pushed open, and infusion, blood collection, and hemodialysis will be performed.

With the indwelling needle assembly 10 having the above-mentioned structure, the pressing operation of the operating button 28 is blocked by the lock part before sticking and before the operation of pushing out the outer needle hub 18 forward in the direction of sticking. Therefore, it is possible to avoid a trouble that the inner needle 22 is housed in the housing 26 due to unintentional contact by the user with the operating button 28 before sticking or the like, for example. In particular, since the lock part is unlocked (the pressing operation of the operating button 28 is enabled) by the operation typically performed when the indwelling needle assembly is stuck, that is, pushing out the outer needle hub 18 forward in the direction of sticking. Thus, no special operation is performed during unlocking the lock part, thereby improving operability.

Additionally, in the present practical embodiment, the projecting lock piece 118 provided to the operating button 28 is inserted into the lock groove 42 provided to the outer needle hub 18, so as to prevent the relative rotation of the outer needle hub 18 and the operating button 28 in the circumferential direction. With this configuration, when the outer needle 16 is delivered to the distal end side, the outer needle hub 18 is prevented from rotating in the circumferential direction, so that the operating force exerted on the outer needle hub 18 is prevented from being spent as a force in the rotation direction of the outer needle hub 18. This makes it possible to efficiently deliver the outer needle 16 to the distal end side.

In the present practical embodiment in particular, the delivery operating part that exerts the operating force for delivering the outer needle 16 to the distal end side is constituted by the outer needle hub 18 that is directly fixed to the proximal end side of the outer needle 16. Thus, the outer needle 16 is delivered to the distal end side with a better transmission efficiency of the operating force.

Moreover, in the present practical embodiment, the support mechanism is provided and the outer needle hub 18 is delivered to the distal end side in a state of being positioned roughly coaxially with respect to the housing 26. Thus, the outer needle 16 is delivered to the distal end side with an even better transmission efficiency of the operating force.

Besides, since the outer needle hub 18 and the operating button 28 are mutually positioned in the circumferential direction, when the indwelling needle assembly 10 is used, the outer needle hub 18 can be prevented from accidentally rotating with the operating button 28 oriented upward. With this configuration, for example, the operating piece 46 for delivering the outer needle hub 18 forward in the direction of sticking can be more reliably positioned in the upper side, and further improvement in operability can be achieved. Moreover, by providing the operating piece 46, when pushing out the outer needle hub 18 forward in the direction of sticking, the risk of contact by the user with the proximal end opening part 38 of the outer needle hub 18 is reduced. Thus, when connecting an external flow path such as a syringe to the proximal end opening part 38, the risk of bacteria or the like being mixed into the body of the patient can be reduced.

Furthermore, in the present practical embodiment, not only the operating button 28 and the operating piece 46 but also the lock part is exposed on the outer circumferential surface and is located on the upper side when the indwelling needle assembly 10 is used. Therefore, it is possible to easily recognize whether or not the pressing operation of the operating button 28 is enabled.

Moreover, in the present practical embodiment, the lock part is constituted by the projecting lock piece 118 provided to the operating button 28 being inserted in the lock groove 42 provided to the outer needle hub 18, so as to prevent the pressing operation of the operating button 28 while exhibiting positioning effect in the circumferential direction. That is, since the lock part is constituted so as to also serve as the circumferential positioning mechanism, the structure can be simplified in compared with the case where the circumferential positioning mechanism is separately provided, for example.

Furthermore, in the present practical embodiment, the lock part is exposed on the outer circumferential surface of the indwelling needle assembly 10. With this configuration, for example, the space inside the outer needle hub 18 can be advantageously obtained, and the arrangement of the hemostasis valve mechanism 48 and the like can be realized more reliably.

Further, in the present practical embodiment, in the initial state, the distal end portion of the inner needle hub 24 (the small-diameter part 66) comprises the insertion part 124, and enters the inside from the proximal end opening part 38 of the outer needle hub 18. With this arrangement, the peripheral wall 32 of the outer needle hub 18 can be sandwiched radially between the projecting lock piece 118 (the contact part 120) of the operating button 28 and the insertion part 124. Therefore, for example, even when an external force in the bending direction is applied to the indwelling needle assembly 10, it is possible to effectively prevent the inner needle hub 24 from slipping out of the outer needle hub 18 or the like.

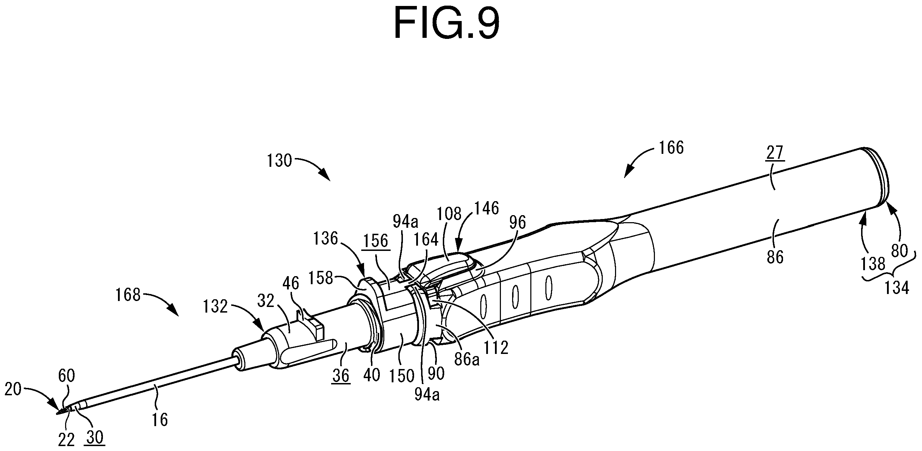

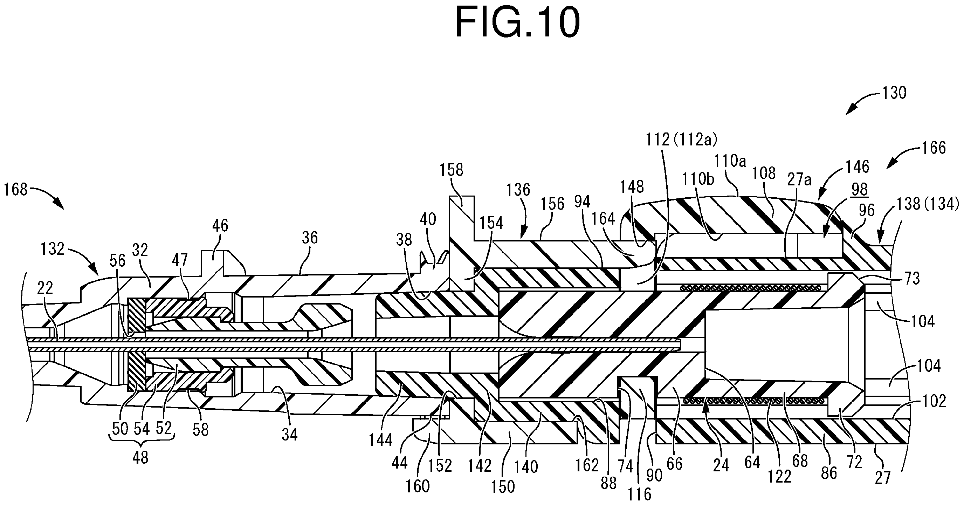

Next, FIGS. 9 and 10 depict an indwelling needle assembly 130 according to a second practical embodiment of the present invention in the initial state. In the present practical embodiment, a slider 136 is provided axially between an outer needle hub 132 and a housing 134. After sticking of the indwelling needle assembly 130, the slider 136 is delivered forward in the direction of sticking so that the outer needle 16 and the outer needle hub 132 are delivered forward in the direction of sticking via the slider 136. That is, in the present practical embodiment, the slider 136 constitutes the delivery operating part that receives the operating force for delivering the outer needle 16 to the distal end side. The components and parts that are substantially identical with those in the preceding first practical embodiment will be assigned like symbols and not described in any detail.

The outer needle hub 132 of the present practical embodiment has an approximately similar structure to that of the outer needle hub (18) of the first practical embodiment except that a lock groove (42) is not provided to the upper side of the flange part 40 provided to the proximal end opening part 38, so that detailed description thereof will be omitted.

Meanwhile, a housing body 138 of the housing 134 of the present practical embodiment includes the tubular peripheral wall 86 extending roughly straight overall, and the inside diameter dimension is reduced at the distal end opening part 88 of the peripheral wall 86. A mating tube part 140 projects to the distal end side from the radially inner edge part of the distal end opening part 88. To the upper side of the outer circumferential surface 27 of the distal end opening part 88 of the peripheral wall 86, the mating groove 94 extending in the axial direction and communicating with the insertion hole 90 is formed.

The mating tube part 140 has a tubular shape that extends roughly straight, and has an outside diameter dimension and an inside diameter dimension smaller than those of the peripheral wall 86. An annular distal end wall 142 projecting to the radially inner side is provided at the distal end of the mating tube part 140, and an insertion tube part 144 projects from the radially inner edge part of the distal end wall 142 toward the distal end side. That is, the housing body 138 of the present practical embodiment includes the peripheral wall 86, the mating tube part 140, and the insertion tube part 144, each having a roughly tubular shape, and has a roughly stepped tubular shape whose outside diameter dimension and inside diameter dimension decrease in a stepwise manner toward the distal end side. The distal end portion of the insertion tube part 144 has a tapered shape that gradually decreases in diameter toward the distal end side, corresponding to the proximal end opening part 38 of the outer needle hub 18.

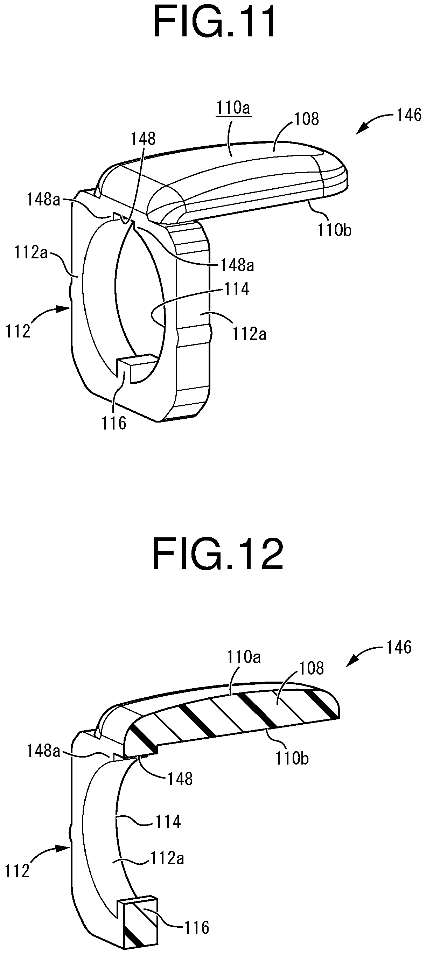

Besides, an operating button 146 as shown in FIGS. 11 and 12 is attached to the housing 134 of the present practical embodiment. Specifically, the operating button 146 of the present practical embodiment is not provided with the projecting lock piece (118) as in the first practical embodiment, and instead, a lock groove 148 opening to the radially inner side and serving as a positioning concave part is provided to the upper part of the inner hole 114. The lock groove 148 has a generally rectangular cross-sectional shape, and is formed across the entire length of the annular part 112 in the thickness direction (the axial direction). That is, the lock groove 148 is open to the distal end surface and the proximal end surface of the annular part 112. Further, the width dimension of the lock groove 148 is roughly equal to the width dimension of the mating groove 94 provided to the housing body 138. In the initial state shown in FIGS. 9 and 10, the annular part 112 of the operating button 146 is inserted in the insertion hole 90 of the housing body 138, so that the mating groove 94 and the lock groove 148 communicate with each other in the axial direction.

Here, the slider 136 includes a tubular wall 150 having a roughly tubular shape overall, and has a predetermined axial dimension. An annular contact wall 154 projecting to the radially inner side is formed in a distal end opening part 152 of the tubular wall 150. The inside diameter dimensions of the tubular wall 150 and the contact wall 154 are roughly equal to or slightly larger than the outside diameter dimensions of the mating tube part 140 and the insertion tube part 144 in the housing body 138, respectively. In particular, the inside diameter dimension of the contact wall 154 is roughly equal to the inside diameter dimension of the proximal end opening part 38 of the outer needle hub 132.

Additionally, on an outer circumferential surface 156 of the distal end opening part 152 of the slider 136, an operating piece 158 projecting to the radially outer side (the upper side) and serving as a projecting delivery piece is provided in a part of the circumference (the upper side in FIG. 10). In the present practical embodiment, the operating piece 158 has a roughly trapezoidal plate shape, and projects upward with a projecting dimension larger than that of the operating piece 46 provided to the outer needle hub 132.

Moreover, in the distal end opening part 152 of the slider 136, a positioning protrusion 160 projecting from the distal end of the tubular wall 150 to the distal end side is formed on the diametrically opposite side to the side where the operating piece 158 is provided (namely, the lower side).

Meanwhile, in a proximal end opening part 162 of the slider 136, a projecting lock piece 164 serving as a positioning convex part and projecting from the proximal end of the tubular wall 150 to the proximal end side is formed at the same position as the operating piece 158 on the circumference (the upper side). The projecting lock piece 164 has a projecting piece shape with a generally rectangular cross section, and the width dimension of the projecting lock piece 164 is approximately equal to or slightly smaller than the width dimensions of the mating groove 94 provided to the housing body 138 and the lock groove 148 provided to the operating button 146.

An inner needle unit 166 of the present practical embodiment is constituted by including the housing 134, the operating button 146, the slider 136, the inner needle 22, and the inner needle hub 24 having the above-mentioned structures. That is, the annular part 112 of the operating button 146 is inserted in the insertion hole 90 of the housing body 138 so that the operating button 146 is attached to the housing body 138, while the inner needle 22 and the inner needle hub 24 are inserted from the proximal end opening part (78) of the housing body 138, and the proximal end opening part (78) is closed off by the cap 80, so that the inner needle 22 and the inner needle hub 24 are housed inside the housing 134.

In the initial state shown in FIGS. 9 and 10, the inner needle 22 and the inner needle hub 24 extend to the distal end side through the inner hole 114 of the operating button 146. That is, the distal end surface of the inner needle hub 24 (the distal end surface of the small-diameter part 66) is in contact with the proximal end surface of the distal end wall 142 provided at the distal end portion of the housing body 138, while the engaging convex part 116 projecting from the lower side of the annular part 112 of the operating button 146 to the radially inner side (upward) is inserted in and engaged with the engaging concave part 74 that opens to the lower side of the inner needle hub 24. In such an assembled state, the coil spring 122, which serves as an urging member and is arranged axially between the flange-shaped part 72 of the inner needle hub 24 and the inner projections 106, 106, 106, 106 projecting to the radially inner side of the housing 134, is in a compressed state, and the inner needle hub 24 is subjected to an urging force toward the proximal end side with respect to the housing 134 based on the elastic recovering deformation of the coil spring 122. Besides, the engaging convex part 116 engages with the engaging concave part 74, so as to block movement of the inner needle hub 24 to the proximal end side according to the urging force.

The slider 136 is attached to the distal end portion of the housing 134 as described above. That is, the tubular wall 150 of the slider 136 is overlapped and externally fitted onto the mating tube part 140 of the housing body 138, and the contact wall 154 of the slider 136 is in contact with the distal end surface of the distal end wall 142 while being overlapped and externally fitted onto the insertion tube part 144. In other words, the slider 136 is overlapped and externally fitted onto the distal end portion of the housing body 138, and the insertion tube part 144 of the housing body 138 projects from the distal end opening part 152 of the slider 136. With this configuration, the center axis of the housing 134 and the center axis of the slider 136 are aligned with each other and are roughly coaxially supported. That is, a support mechanism that positions and supports the slider 136 roughly coaxially with respect to the housing 134 is constituted by the slider 136 being overlapped and externally fitted onto the distal end portion of the housing body 138 (the external fit of the tubular wall 150 onto the mating tube part 140, and the external fit of the contact wall 154 onto the insertion tube part 144). The slider 136 is movable in the axial direction in a state of being positioned roughly coaxially with respect to the housing 134 by such a support mechanism.

Here, the projecting lock piece 164 projecting from the slider 136 to the proximal end side is inserted in the mating groove 94 provided to the housing body 138 and the lock groove 148 provided to the operating button 146. That is, the projecting lock piece 164 is inserted from the radially inner side into the lock groove 148 that opens to the radially inner side. By the groove upper wall surface of the lock groove 148 and the upper surface of the projecting lock piece 164 being in contact with each other in the axis-perpendicular direction of the housing 134, namely, in the vertical direction, downward movement (pressing operation) of the operating button 146 is blocked. Therefore, in the present practical embodiment, the projecting lock piece 164 is inserted into the lock groove 148 from the radially inner side so as to constitute a lock part that blocks the pressing operation of the operating button 146.

Additionally, similarly to the first practical embodiment, the annular part 112 of the operating button 146 is inserted into the insertion hole 90 of the housing body 138, and/or the base part 108 of the operating button 146 is arranged in the arrangement area 98 provided to the outer circumferential surface 27 of the housing 134, so that the housing 134 and the operating button 146 are nonrotatable relative to each other in the circumferential direction. Besides, in the annular part 112 of the operating button 146, the projecting lock piece 164 is in contact with widthwise opposite portions 148a, 148a sandwiching the lock groove 148 in the circumferential direction, thereby preventing relative rotation of the operating button 146 and the slider 136 in the circumferential direction (the operating button 146 and the slider 136 are positioned relative to each other in the circumferential direction). Further, the circumferentially opposite portions 94a, 94a sandwiching the mating groove 94 of the distal end opening part 88 of the peripheral wall 86 and the projecting lock piece 164 are in contact with each other in the circumferential direction, thereby preventing relative rotation of the slider 136 and the housing 134 in the circumferential direction is prevented. Also, similarly to the first practical embodiment, the inner needle 22 and the inner needle hub 24 are nonrotatable with respect to the housing 134 in the circumferential direction. With these configurations, in the present practical embodiment, all the members constituting the inner needle unit 166 (the inner needle 22, the inner needle hub 24, the housing 134, the slider 136, and the operating button 146) are nonrotatable relative to one another.

That is, in the present practical embodiment, one of the positioning mechanisms for positioning the slider 136 with respect to the housing 134 in the circumferential direction includes the projecting lock piece 164 and the lock groove 148. Besides, the housing 134 and the operating button 146 are nonrotatable relative to each other in the circumferential direction. Thus, by the projecting lock piece 164 being inserted into the mating groove 94 as well, the slider 136 is positioned with respect to the operating button 146 in the circumferential direction via the housing 134. Therefore, in the present practical embodiment, another one of the positioning mechanisms for positioning the slider 136 with respect to the housing 134 in the circumferential direction includes the relative rotation prevention mechanism in the circumferential direction between the housing 134 and the operating button 146, as well as the projecting lock piece 164 and the mating groove 94.

An outer needle unit 168 is attached to the said inner needle unit 166, thereby constituting the indwelling needle assembly 130 of the present practical embodiment. That is, the inner needle 22 is inserted from the proximal end opening part 38 of the outer needle hub 132, penetrates the hemostasis valve mechanism 48 and the outer needle 16, and the needle tip 20 of the inner needle 22 projects from the outer needle 16. Besides, the distal end opening part 152 of the slider 136 is axially continuous with the proximal end opening part 38 of the outer needle hub 132, and the insertion tube part 144 of the housing body 138 projecting from the distal end opening part 152 of the slider 136 to the distal end side is inserted in the inside of the outer needle hub 132 through the proximal end opening part 38 of the outer needle hub 132.

In the present practical embodiment, since the distal end portion of the insertion tube part 144 has a tapered shape corresponding to the proximal end opening part 38 of the outer needle hub 132, the outer circumferential surface of the insertion tube part 144 and the inner circumferential surface 34 of the outer needle hub 132 is in contact with each other with almost no gap. In particular, the insertion tube part 144 is inserted into the proximal end opening part 38 of the outer needle hub 132 with a certain degree of mating force, thereby making it also possible to prevent the outer needle unit 168 from accidentally falling out of the inner needle unit 166.

Moreover, the positioning protrusion 160 projecting from the lower side of the slider 136 to the distal end side is inserted in the positioning concave groove 44 provided to the lower side of the flange part 40 of the outer needle hub 132. In other words, in the proximal end opening part 38 of the outer needle hub 132, the peripheral wall 32 of the outer needle hub 132 is sandwiched radially between the insertion tube part 144 of the housing body 138 and the positioning protrusion 160 of the slider 136. With this configuration, relative rotation of the slider 136 and the outer needle hub 132, namely, the inner needle unit 166 and the outer needle unit 168 in the circumferential direction is prevented. It would also be acceptable to prevent the relative rotation of the inner needle unit 166 and the outer needle unit 168 in the circumferential direction by, for example, the frictional force of contact between the inner circumferential surface 34 of the outer needle hub 132 and the outer circumferential surface of the insertion tube part 144. Further, it would also be possible to provide the slider 136 with a projecting part having the same structure as the projecting lock piece 118 in the first practical embodiment, and to provide the outer needle hub 132 with a notch having the same structure as the lock groove 42 in the first practical embodiment. Such an engagement structure between the projecting part and the notch may be adopted in place of, or in combination with the engagement between the positioning protrusion 160 and the positioning concave groove 44, thereby preventing the relative rotation of the slider 136 and the outer needle hub 132 in the circumferential direction.

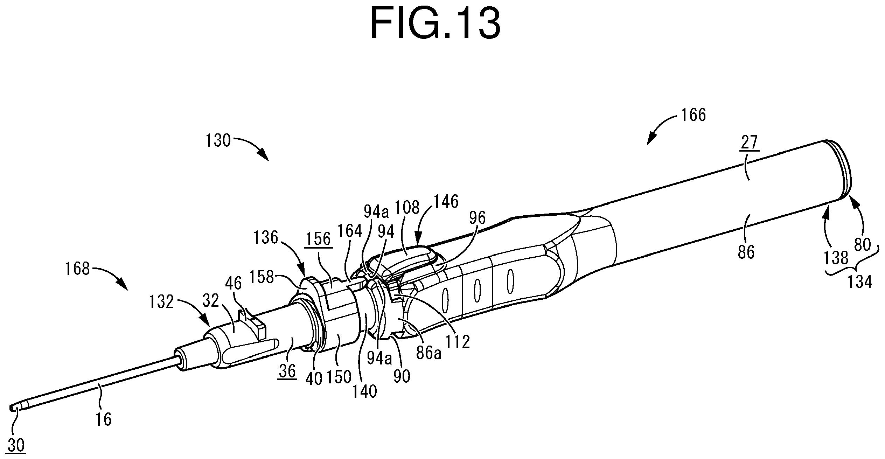

The indwelling needle assembly 130 having the above-mentioned structure is stuck with the inner needle 22 inserted in the outer needle 16 as shown in FIGS. 9 and 10. Then, as shown in FIG. 13, the user puts a finger on the operating piece 158 of the slider 136 and delivers the slider 136 forward in the direction of sticking. By so doing, the outer needle 16 and the outer needle hub 132 are delivered forward in the direction of sticking together with the slider 136, and the outer needle 16 is pushed deeper into the blood vessel of the patient.

The movement of the slider 136 forward in the direction of sticking may be regulated by, for example, the following slip-out prevention mechanism. Specifically, such a dropout prevention mechanism can be constituted by, for example, a structure in which a concave and a convex are provided between the overlapped surfaces of the slider 136 and the housing body 138, and after the slider 136 moves forward by a predetermined distance with respect to the housing body 138, the concave and the convex are mutually mated in a concave and convex manner. With this structure, the amount of movement of the slider 136 forward in the direction of sticking is defined (namely, the slider 136 can move to the distal end side by a predetermined amount), and the slider 136 is effectively prevented from slipping out of the housing body 138, and at the same time, the amount of delivery of the outer needle 16 to the distal end side can also be defined.

Besides, by pushing out the slider 136 forward in the direction of sticking in this way, the projecting lock piece 164 is detached from the lock groove 148, and the contact between the groove upper wall surface of the lock groove 148 provided to the operating button 146 and the projecting lock piece 164 provided to the slider 136 is released. By so doing, the downward movement (the pressing operation) of the operating button 146 is allowed.

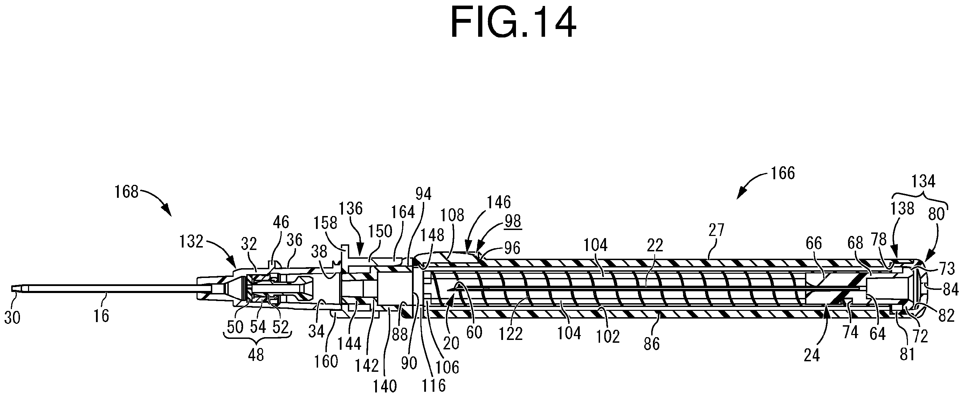

Then, as shown in FIG. 14, through the pressing operation of the operating button 146, the engagement between the engaging convex part 116 and the engaging concave part 74 is released, and the inner needle hub 24 moves to the proximal end side of the housing 134 according to the urging force. By so doing, the outer needle unit 168 is indwelled in the skin of the patient, and the needle tip 20 of the inner needle 22 is protected by the housing 134, so that the inner needle unit 166 can be safely discarded or the like.

The indwelling needle assembly 130 of the present practical embodiment having the above-mentioned structure is also able to exhibit the same effect as that of the first practical embodiment.

In the present practical embodiment in particular, the slider 136 is provided axially between the outer needle hub 132 and the housing 134. Since the slider 136 has a predetermined axial dimension, and the amount of projection of the operating piece 158 is relatively large, it is easy to place a finger on the slider 136 and deliver the slider 136 forward in the direction of sticking.
Moreover, since the operating button 146 and the slider 136 are mutually positioned in the circumferential direction, the operating piece 158 provided to the slider 136 can be stably oriented upward, thereby more easily performing the delivery operation of the slider 136 forward in the direction of sticking.

Although the practical embodiments of the present invention have been described above, the present invention is not limitedly interpreted based on the specific description in the practical embodiments, but may be embodied with various changes, modifications and improvements which may occur to those skilled in the art.

For example, in the first practical embodiment, the circumferential positioning of the operating button 28 and the outer needle hub 18 is realized by the projecting lock piece 118 (particularly the contact part 120) being mated with the lock groove 42, and by the positioning protrusion 100 being mated with the positioning concave groove 44. However, one of such mating structures may be adopted for the circumferential positioning. That is, in the case where the operating button 28 is provided nonrotatably with respect to the housing 26 in the circumferential direction, and the positioning protrusion 100 and the positioning concave groove 44 are provided, the projecting lock piece 118 and the lock groove 42 do not have to exhibit the circumferential positioning effect since the outer needle hub 18 and the operating button 28 are positioned in the circumferential direction via the housing 26. Alternatively, if the projecting lock piece 118 and the lock groove 42 exhibit the circumferential positioning effect, the positioning protrusion 100 and the positioning concave groove 44 do not have to be provided. That is, the lock part (the projecting lock piece 118 and the lock groove 42 in the first practical embodiment) does not need to additionally have a circumferential positioning function. Besides, in the second practical embodiment, the relative rotation of the outer needle unit 168 and the inner needle unit 166 in the circumferential direction is prevented by the positioning protrusion 160 being mated with the positioning concave groove 44, and by the insertion tube part 144 being inserted in the outer needle hub 132, thereby making it possible to stably slide the slider 136 and the outer needle hub 132 toward the distal end. However, such a structure is not essential, but the outer needle unit 168 and the inner needle unit 166 may rotate relative to each other in the circumferential direction. That is, the positioning protrusion 160 and the positioning concave groove 44 are not essential, and the inner circumferential surface 34 of the outer needle hub 132 and the outer circumferential surface of the insertion tube part 144 do not have to be in contact with each other but a gap may be provided therebetween.

Additionally, in the second practical embodiment, when the inner needle 22 and the inner needle hub 24 move to the proximal end side with respect to the housing 134, the needle tip 20 of the inner needle 22 is configured to be covered and protected by the housing 134. However, the present invention is not limited to such a mode. That is, when the inner needle 22 moves to the proximal end side with respect to the housing 134, the needle tip 20 may project from the housing 134, and the needle tip 20 may be covered and protected by the slider 136 that has moved to the distal end side with respect to the housing 134. In the first and second practical embodiments, the needle tip 20 of the inner needle 22 after being pulled out is protected by the housing 26, 134, and the inner needle unit 14, 166 is configured to be safely discarded or the like. However, the needle tip 20 of the inner needle 22 after being pulled out may project from the housing 26, 134 or the slider 136.

Moreover, in the second practical embodiment, the operating pieces 46, 158 are provided to both the outer needle hub 132 and the slider 136. However, in the case where the operating piece 158 is provided to the slider 136, the operating piece 46 does not need to be provided to the outer needle hub 132. By providing the operating pieces 46, 158 to both the outer needle hub 132 and the slider 136 as in the second practical embodiment, it would also be possible to deliver the slider 136 forward in the direction of sticking by using the operating piece 158, and then to additionally deliver the outer needle hub 132 forward in the direction of sticking by using the operating piece 46. However, these operating pieces 46, 158 are not essential in the present invention, but the outer circumferential surface 36 of the outer needle hub 18, 132 and the outer circumferential surface 156 of the slider 136 may be directly operated.

Furthermore, in the first practical embodiment, as a support mechanism for positioning and supporting the outer needle hub 18 and the housing 26 roughly coaxially, illustrated is a mode in which the outer circumferential surface of the insertion part 124 is in contact with the inner circumferential surface 34 of the proximal end opening part 38 of the outer needle hub 18 with almost no gap. However, the present invention is not limited to such a mode. That is, for example, the support mechanism may be constituted by clasping the peripheral wall 32 of the outer needle hub 18 in the vertical direction between the contact part 120 of the projecting lock piece 118 and the positioning protrusion 100. Alternatively, the support mechanism may be constituted by sandwiching and clasping the peripheral wall 32 of the outer needle hub 18 radially between the insertion part 124, which is the distal end portion of the inner needle hub 24 that enters the inside of the outer needle hub 18, and the contact part 120 and/or the positioning protrusion 100. Alternatively, the support mechanism may be constituted by providing portions to be mated in a concave and convex manner between the contact part 120 and/or the positioning protrusion 100 and the outer circumferential surface 36 of the outer needle hub 18, or by providing portions to be mated in a concave and convex manner between the insertion part 124 and the inner circumferential surface 34 of the outer needle hub 18. As the support mechanism, two or more of the above-exemplified modes may be adopted in combination.

Besides, in the preceding practical embodiment, the lock part (the projecting lock piece 118 and the lock groove 42 in the first practical embodiment, and the projecting lock piece 164 and the lock groove 148 in the second practical embodiment) is provided so as to be exposed on the outer circumferential surface of the indwelling needle assembly 10, 130. However, the lock part may be provided inside the indwelling needle assembly 10, 130, namely, inside the outer needle hub 18, 132, or inside the slider 136. Further, the lock part and the operating button 28, 146 do not need to be provided at the same position on the circumference (the upper side). For example, the lock part may be provided on the diametrically opposite side with respect to the operating button 28, 146 with the inner needle 22 sandwiched therebetween.

Moreover, in the first practical embodiment, the outer needle hub 18 is provided with the lock groove 42 (the positioning concave part) and the operating button 28 is provided with the projecting lock piece 118 (the positioning convex part). However, it would also be acceptable that, for example, the operating button 28 is provided with a positioning concave part and the outer needle hub 18 is provided with a positioning convex part projecting to the proximal end side, and by these positioning concave and convex parts being mated in a concave and convex manner and being in contact in the vertical direction, the movement of the operating button 28 in the direction of pressing is blocked. Furthermore, in the second practical embodiment, the slider 136 is provided with the projecting lock piece 164 (the positioning convex part) and the operating button 146 is provided with the lock groove 148 (the positioning concave part). However, it would also be acceptable that, for example, the slider 136 is provided with a positioning concave part and the operating button 146 is provided with a positioning convex part projecting to the distal end side, and by these positioning concave and convex parts being mated in a concave and convex manner and being in contact in the vertical direction, the movement of the operating button 28 in the direction of pressing is blocked.

Additionally, in the preceding practical embodiment, the coil spring 122 is adopted as the urging member. However, it would also be possible to adopt those utilizing elasticity such as a rubber and a leaf spring, as well as those utilizing magnetic force such as a magnet.

### KEYS TO SYMBOLS

10, 130: indwelling needle assembly, 16: outer needle, 18, 132: outer needle hub, 22: inner needle, 24: inner needle hub, 26, 134: housing, 27: outer circumferential surface of housing, 28, 146: operating button (operating member), 32: peripheral wall of outer needle hub, 36: outer circumferential surface of outer needle hub, 38: proximal end opening part of outer needle hub, 40: flange part, 42, 148: lock groove (positioning concave part, positioning mechanism, lock part), 44: positioning concave groove (positioning mechanism), 46, 158: operating piece (projecting delivery piece), 50: hemostasis valve, 80: cap, 94: mating groove (positioning mechanism), 100, 160: positioning protrusion (positioning mechanism), 116: engaging convex part (engaging part), 118, 164: projecting lock piece (positioning convex part, positioning mechanism, lock part), 122: coil spring (urging member), 124: insertion part, 136: slider, 156: outer circumferential surface of slider

## Claims

1. An indwelling needle assembly including: a housing; an outer needle arranged so as to project to a distal end side of the housing; and an inner needle inserted through the outer needle, the inner needle being configured to be housed in the housing by means of an urging member through pressing operation of an operating member that is provided so as to be exposed on an outer circumferential surface of the housing, wherein
the indwelling needle assembly further includes:
a delivery operating part configured to receive an operating force that delivers the outer needle from the housing to the distal end side;
a lock part provided with respect to the delivery operating part, the lock part blocking movement of the operating member in a direction of pressing while releasing blocking of the movement through delivery of the outer needle by means of the delivery operating part; and
a positioning mechanism that positions the delivery operating part with respect to the operating member in a circumferential direction of the housing.

2. The indwelling needle assembly according to claim 1, wherein the delivery operating part is constituted by an outer needle hub provided on a proximal end side of the outer needle.

3. The indwelling needle assembly according to claim 1, wherein the delivery operating part is constituted by a slider arranged between an outer needle hub provided on a proximal end side of the outer needle and the housing.

4. The indwelling needle assembly according to any one of claims 1-3, wherein a support mechanism is provided, the support mechanism allowing delivery of the delivery operating part to the distal end side while coaxially positioning the delivery operating part with respect to the housing, and
the lock part is constituted by a structure in which the operating member and the delivery operating part are in contact with each other in an axis-perpendicular direction of the housing.

5. The indwelling needle assembly according to any one of claims 1-4, wherein the positioning mechanism is constituted by a structure in which the operating member and the delivery operating part are in contact with each other in the circumferential direction of the housing.

6. The indwelling needle assembly according to any one of claims 1-5, wherein one of the operating member and the delivery operating part includes a positioning concave part opening toward the other of the operating member and the delivery operating part, and the other of the operating member and the delivery operating part includes a positioning convex part projecting toward the one of the operating member and the delivery operating part, and
the positioning convex part is inserted in the positioning concave part, and the positioning convex part is configured to be detached from the positioning concave part through delivery of the outer needle by means of the delivery operating part so as to constitute the lock part and the positioning mechanism.

7. The indwelling needle assembly according to any one of claims 1-6, wherein the delivery operating part includes a projecting delivery piece configured to directly receive the operating force that delivers the outer needle from the housing to the distal end side, the projecting delivery piece projecting on an outer circumference of the delivery operating part at a position corresponding to the operating member of the housing in the circumferential direction.

8. The indwelling needle assembly according to any one of claims 1-7, wherein
an inner needle hub provided on a proximal end side of the inner needle is urged to the proximal end side by the urging member and arranged within the housing,
an engaging part is provided to the operating member on a distal end side of the direction of pressing, the engaging part projecting into the housing and engaging with the inner needle hub so as to block movement of the inner needle hub due to the urging member, and
through the movement of the operating member in the direction of pressing, the engaging part is configured to move outward from the housing so as to release engagement with the inner needle hub.

9. An indwelling needle assembly including: a housing; an outer needle arranged so as to project to a distal end side of the housing; and an inner needle inserted through the outer needle, the inner needle being configured to be housed in the housing by means of an urging member through pressing operation of an operating member that is provided so as to be exposed on an outer circumferential surface of the housing, wherein
an inner needle hub is provided on a proximal end side of the inner needle, a cap is arranged on the proximal end side of the housing, and a moving end of the inner needle to the proximal end side by means of the urging member when the inner needle is housed in the housing is defined by the inner needle hub coming into contact with the cap, while at least one of following structures (i)-(x) is adopted as a reducing mechanism of a striking noise of the inner needle hub against the cap:
(i) a structure adopting the cap comprising an elastic material exhibiting a shock absorbing action due to elastic deformation or viscoelastic deformation during contact of the inner needle hub;
(ii) a structure in which a cushioning material that exhibits a shock absorbing action is arranged on a contact part between the inner needle hub and the cap;
(iii) a structure in which the cap, with which the inner needle hub comes into contact, is attached to a proximal end opening part of a housing body in a non-adhesive way with a protrusion for press-fit mating so as to reduce transmission of the striking noise of the inner needle hub against the cap to the housing body;
(iv) a structure in which a tubular-shaped inner circumferential surface of the cap, with which an outer circumferential portion on the proximal end side of the inner needle hub comes into contact, comprises an inclined surface that is constricted in diameter backward so as to gradually increase a contact force of the inner needle hub against the cap;
(v) a structure in which a contact site between the inner needle hub and the cap has a substantially linear shape extending in the circumferential direction and a width dimension of a contact surface is reduced so as to avoid a large striking noise due to striking by a flat surface;
(vi) a structure in which the inner needle hub and the cap are configured to come into contact with each other by contact portions extending in the circumferential direction, and at least one of the contact portions of the inner needle hub and the cap includes a notch or a recess provided partially in the circumferential direction and the contact site between the inner needle hub and the cap is made partial in the circumferential direction so as to exhibit a cushioning action due to deformation of the contact site and/or a striking noise reducing action due to decrease in a contact area;
(vii) a structure in which a rigidity of a proximal end portion of the inner needle hub that comes into contact with the cap is reduced by an inner circumferential surface that gradually expands toward a side of the cap and/or an outer circumferential surface that gradually tapers toward the side of the cap so as to exhibit a cushioning action due to striking deformation of the contact portion of the inner needle hub against the cap;
(viii) a structure in which a tube-shaped projecting part is provided to the cap with which the inner needle hub comes into contact, and the tube-shaped projecting part is fitted in the proximal end opening part of the housing body while a proximal side end of the inner needle hub is configured to be inserted into the tube-shaped projecting part so as to avoid a direct contact of the inner needle hub with the housing body during striking of the inner needle hub against the cap and to prevent generation of a noise caused by the contact with the housing body which is rigid;
(ix) a structure in which a coil spring is adopted as the urging member, and a natural length of the coil spring is set smaller than a movement distance of the inner needle hub in an axial direction so as to exhibit a striking noise reducing action due to a tensile force of the coil spring during striking between the inner needle hub and the cap; and
(x) a structure in which a hemostasis valve is attached to an outer needle hub provided on the proximal end side of the outer needle, the inner needle is arranged through the hemostasis valve, and a compression force is exerted on an outer circumferential surface of the hemostasis valve such that the hemostasis valve applies an urging force to the inner needle due to the compression force.
